# EUROPEAN PATENT SPECIFICATION

(11) **EP 3 535 402 B1**
(45) Date of publication and mention of the grant of the patent: **07.06.2023**
(21) Application number: 17800454.5
(22) Date of filing: 03.11.2017
(51) Int. Cl.: C12N 15/86, A61K 48/00

(54) **LENT-ON-PLUS SYSTEM FOR CONDITIONAL EXPRESSION IN HUMAN STEM CELLS**
LENT-ON-PLUS-SYSTEM ZUR BEDINGTEN EXPRESSION IN MENSCHLICHEN STAMMZELLEN
SYSTÈME LENT-ON-PLUS POUR L'EXPRESSION CONDITIONNELLE DANS DES CELLULES SOUCHES HUMAINES

(30) Priority: 04.11.2016 ES 201631406
(43) Date of publication of application: 11.09.2019
(73) Proprietor: Fundación Pública Andaluza Progreso Y Salud, 41092 Sevilla (ES)
(72) Inventor: MARTÍN MOLINA, Francisco, 41092 Sevilla (ES); BENABDELLAH ELKHLANJI, Karim, 41092 Sevilla (ES); MUÑOZ FERNÁNDEZ, Pilar, 41092 Sevilla (ES); COBO PULIDO, Marién, 41092 Sevilla (ES)
(74) Representative: Hoffmann Eitle
(86) International application number: PCT/EP2017/078246
(87) International publication number: WO 2018/083274

(56) References cited:
- WO-A1-2012/156535
- WO-A1-2014/140218
- KARIM BENABDELLAH ET AL: "Development of an All-in-One Lentiviral Vector System Based on the Original TetR for the Easy Generation of Tet-ON Cell Lines", PLOS ONE, vol. 6, no. 8, 1 January 2011 (2011-01-01) , pages e23734-e23734, XP055038075, ISSN: 1932-6203, DOI: 10.1371/journal.pone.0023734 cited in the application
- KARIM BENABDELLAH ET AL: "A Chimeric HS4-SAR Insulator (IS2) That Prevents Silencing and Enhances Expression of Lentiviral Vectors in Pluripotent Stem Cells", PLOS ONE, vol. 9, no. 1, 6 January 2014 (2014-01-06) , page e84268, XP055127119, DOI: 10.1371/journal.pone.0084268 cited in the application
- PLUTA KRZYSZTOF ET AL: "TIGHT CONTROL OF TRANSGENE EXPRESSION BY LENTIVIRUS VECTORS CONTAINING SECOND-GENERATION TETRACYCLINE-RESPONSIVE PROMOTERS", JOURNAL OF GENE MEDI, JOHN WILEY & SONS, INC, US, vol. 7, no. 6, 1 June 2005 (2005-06-01), pages 803-817, XP009078421, ISSN: 1099-498X, DOI: 10.1002/JGM.712
- VAYSSE L ET AL: "Development of a Self-assembling Nuclear Targeting Vector System Based on the Tetracycline Repressor Protein", JOURNAL OF BIOLOGICAL CHEMISTRY, AMERICAN SOCIETY FOR BIOCHEMISTRY AND MOLECULAR BIOLOGY, vol. 279, no. 7, 13 February 2004 (2004-02-13), pages 5555-5564, XP002509635, ISSN: 0021-9258, DOI: 10.1074/JBC.M311894200 [retrieved on 2003-11-07]
- KARIM BENABDELLAH ET AL: "Lent-On-Plus Lentiviral vectors for conditional expression in human stem cells", SCIENTIFIC REPORTS, vol. 6, no. 1, 17 November 2016 (2016-11-17), XP055443844, DOI: 10.1038/srep37289

## Description

### FIELD OF THE INVENTION

The present invention refers to improved gene transfer vectors for gene therapy and basic research. Particularly, the present invention relates to new DNA sequences capable of improve expression regulation and avoid silencing of gene transfer vectors (viral or non-viral). More particularly, the present invention refers to new combinations of DNA sequences capable of improve expression regulation and avoid silencing of gene transfer vectors (viral or non-viral) in mammalian stem cells. More particularly, the present invention refers to new combinations of DNA sequences capable of improve expression regulation and avoid silencing of gene transfer vectors (viral or non-viral) for gene therapy applications.

### BACKGROUND OF THE INVENTION

Technologies allowing conditional transgene expression in human stem cells are fundamental not only to study gene function but also as potential tools for gene therapy. Gene therapy has been proposed to be a promising tool to treat diseases. The aim of gene therapy is to introduce therapeutic genes into target cells, leading to efficient, stable and/or regulated expression of the therapeutic molecules and without affecting the normal physiology of the target cell.

However, gene transfer vectors (the tools used to transfer the gene into the cells) are confronted with several obstacles such as:
- Gene silencing: most vectors lose their expression capacity due to epigenetic modifications.
- Variegation: the expression patterns of integrative vectors such as retrovirus vary depending on the site of integration, making it difficult to control the expression of the transgene.
- Finally, the integrative vectors affect the expression pattern of the target cell due to the presence of several elements (enhancers, cryptic splice donor and acceptors, polyadenylation sites) that influence the normal expression of genes located near the integration sites.

Several approaches have been proposed to try to solve these obstacles, such as the use of human promoters (Hong, S. et al. Functional analysis of various promoters in lentiviral vectors at different stages of in vitro differentiation of mouse embryonic stem cells. Mol Ther (2007); Kita-Matsuo, H. et al. Lentiviral vectors and protocols for creation of stable hESC lines for fluorescent tracking and drug resistance selection of cardiomyocytes. PLoS ONE (2009)), or of promoters with low methylation levels, or the incorporation of insulators (Benabdellah, K. et al., A chimeric HS4-SAR insulator (IS2) that prevents silencing and enhances expression of lentiviral vectors in pluripotent stem cells. PLoS ONE (2014); Aker, M. et al. Extended core sequences from the cHS4 insulator are necessary for protecting retroviral vectors from silencing position effects. Hum Gene Ther (2007); Pfaff, N. et al. A ubiquitous chromatin opening element prevents transgene silencing in pluripotent stem cells and their differentiated progeny. Stem Cells (2013)) in order to avoid the deleterious effects of the transgene over the host chromatin and viceversa. The insulators are based on naturally occurring DNA elements that form functional boundaries between adjacent chromatin domains and play a role in shielding certain genes from other regulatory domains present on its proximity. However, each of these strategies alone is not sufficient to completely solve the obstacles.

In addition, the target cell also dictates the stability of transgene expression and the potential genotoxicity of the integrative vector. In this sense, transgene expression on stem and primary cells are generally more prompt to gene silencing than on differentiated and/or immortalized cell lines.

Tetracycline-regulated gene expression systems (Tet-On or Tet-Off) have been used for conditional gene expression in most stem cells types including human embryonic stem cells (hESCs) (Ormsbee Golden, B.D. et al., Sox2 expression is regulated by a negative feedback loop in embryonic stem cells that involves AKT signaling and FoxO1. PLoS ONE (2013); Vieyra, D.S. & Goodell, M.A. Pluripotentiality and conditional transgene regulation in human embryonic stem cells expressing insulated tetracycline-ON transactivator. Stem Cells (2007); Zhou, B.Y. et al. Inducible and reversible transgene expression in human stem cells after efficient and stable gene transfer. Stem Cells (2007); Xia, X. et al., In vitro- and in vivo-induced transgene expression in human embryonic stem cells and derivatives. Stem Cells (2008)), induced pluripotent stem cells (iPSCs) (Qian, K. et al. A simple and efficient system for regulating gene expression in human pluripotent stem cells and derivatives. Stem Cells (2014)) and mesenchymal stromal cells (hMSCs) (Pan, Z.M. et al. Treatment of Femoral Head Necrosis With Bone Marrow Mesenchymal Stem Cells Expressing Inducible Hepatocyte Growth Factor. Am J Ther (2015); Moriyama, H. et al. Tightly regulated and homogeneous transgene expression in human adipose-derived mesenchymal stem cells by lentivirus with tet-off system. PLoS ONE (2013); Yang, W.H. et al. Regulated expression of lentivirus-mediated GDNF in human bone marrow-derived mesenchymal stem cells and its neuroprotection on dopaminergic cells in vitro. PLoS ONE (2013)).

However, most tetracycline-regulated systems require a tetracycline-dependent-transactivator containing the activating domain of the herpes virus simplex viral protein 16 *(VP-16)* linked to the TetR repressor (tTA for Tet-Off or rtTA for Tet-On systems). The presence of this transactivating domain can make these proteins toxic due to the sequestration of transcription factors required for cell growth (squelching) (Sisson, T.H. et al. Expression of the reverse tetracycline-transactivator gene causes emphysema-like changes in mice. Am J Respir Cell Mol Biol (2006); Perl, A.K. et al., Conditional expression of genes in the respiratory epithelium in transgenic mice: cautionary notes and toward building a better mouse trap. Am J Respir Cell Mol Biol (2009)). In addition, binding of the chimeric TetR-VP16 protein to *pseudo-TetO* sites can trans-activate non-target cellular genes (Hack!, H. et al., Tetracycline regulator expression alters the transcriptional program of mammalian cells. PLoS ONE (2010); Han, H.J. et al. Strain background influences neurotoxicity and behavioral abnormalities in mice expressing the tetracycline transactivator. J Neurosci (2012)) causing unpredicted side effects. Similar consequences have also been reported with other transcriptional activators such as the Crerecombinase and its variant CreER (Forni, P.E. et al. High levels of Cre expression in neuronal progenitors cause defects in brain development leading to microencephaly and hydrocephaly. J Neurosci (2006); Higashi, A.Y. et al. Direct hematological toxicity and illegitimate chromosomal recombination caused by the systemic activation of CreERT2. J Immunol (2009); Naiche, L.A. & Papaioannou, V.E. Cre activity causes widespread apoptosis and lethal anemia during embryonic development. Genesis (2007)). Therefore, even though the tTA(rtTA)/tetO and Cre/lox*P* systems are useful tools for conditional transgene expression in stem cells, they have the potential to influence cellular physiology. Another major obstacle for the wider application of most conditional systems is the general requirement of a selection of the cells that have integrated the transgenes, mainly due to the low efficiency of gene delivery methods and the strong silencing of the transgenes. Cells then have to be exposed to additional steps that add potential side effects to the method and clones might be drug-responsive while being able to regulate transgene expression.

The inventors has described an all-in-one regulated lentiviral vector (CEST) based on the original TetR repressor, that does not need the tetracycling-transactivating domain and that allowed the generation of Dox-regulated cell lines, including primary human fibroblasts (HFF) and human MSCs (hMSCs). However, the CEST LVs were not able to efficiently regulate transgene expression in pluripotent stem cells due to silencing of the TetR expression. In addition this system required multiple integrations per cell in order to achieve regulation in HFF and hMSCs, hindering its application for gene therapy applications.

The inventors have also recently developed a chimeric insulator (Is2) that avoid silencing of LVs in pluripotent hESCs during expansion and upon differentiation (Benabdellah, K. et al., A chimeric HS4-SAR insulator (IS2) that prevents silencing and enhances expression of lentiviral vectors in pluripotent stem cells. PLoS ONE (2014 and patent EP13382080.3).

Overall, although significant improvements have been done in the past years to develop efficient transgene systems, additional efforts are needed to improve the existing techniques and to reach higher efficiency of transgene insertion, transgene expression and expression regulation in stem cells.

### BRIEF DESCRIPTION OF THE INVENTION

In the present invention, the inventors have generated a Tet-On all-in-one construct that tightly regulate transgene expression in human stem cells using the original TetR repressor. By using appropriate promoter combinations and shielding the integrative construct with the Is2 insulator, they have constructed the Lent-On-Plus Tet-On system that achieves efficient transgene regulation in human multipotent and pluripotent stem cells. The generation of inducible stem cell lines with the Lent-ON-Plus system did not require selection or cloning, and transgene regulation is maintained after long-term cultured and upon differentiation toward different lineages.

The present invention offers an alternative solution for the problem cited above, of a need to improve transgene expression systems in stem cells. As shown herein, several genetic constructs have been tested on their capacity to induce the expression of reporter gene (eGFP) upon doxycycline stimulation on the context of a CEST Lentiviral Vector (LV) or of a CEET LV. Both vectors are based on the Tet-On system.

The CEST LV contains a CMV-TetO inducible promoter that controls the expression of the reporter gene eGFP. A spleen focus forming virus (SFFV) promoter controls the expression of a TetR gene that encodes a protein based on the TetR repressor. Upon binding to the CMV-TEtO promoter, the TetR protein inhibits the expression of the eGFP reporter gene. In the presence of doxycycline, the TetR protein is no longer able to repress the CMV-TetO promoter.

The CEET vector is similar to the CEST LV except that it contains a human elongation factor-1 alpha (hEF1α) promoter instead of the SFFV to control the expression of the TetR protein.

The efficiency of eGFP expression in the presence and absence of doxycycline is tested for both vectors, in different mammalian host cells, when containing additional sequences: insulator Is2 with or without the nuclear localization signal nI2 associated to the TetR protein.

In 293T cells, the addition of nuclear localization signal (nI2) to the TetR protein in the CEST vector increased the expression of eGFP. Both, the addition of the nI2 and IS2 to the CEET vector improved the fold induction of eGFP upon doxycycline stimuli. This combination of elements (nI2, hEF1α and Is2) also reduced the unspecific expression of eGFP in the absence of doxycycline resulting from leaking. These improvements were obtained in conditions of low multiplicity of infection (MOI) that mainly allow to transducing only one vector copy per cell.

Similarly, in human mesenchymal stem cells (hMSCs), the use of the CEET vector instead of the CEST vector reduced the leaking in a construct already containing the nI2 signal. The addition of the Is2 to this construct also improved the induced expression of the transgene. This tendency was maintained upon long term culture and differentiation. Again, these results were also observed with cells harboring only one vector copy per cell.

In human embryonic stem cells (hESCs), the use of the CEET vector in combination with the nI2 with or without the Is2 allowed to reach low leaking and good transgene expression in the presence of doxycycline. Upon differentiation towards hMSCs and towards hematopoietic stem cells, the low leaking and good expression levels of the previous constructs was maintained. However, the beneficial effect of the Is2 was mainly observed in the differentiated hMSCs.

Taken together, results show that the use of the hEF1 α promoter, instead of by the SFFV promoter, the addition of the nuclear localization signal nI2 to the TetR protein and the addition of the insulator element IS2 allow to reduce the unspecific expression of the transgene due to leaking and to improve its regulation upon doxycycline stimulation in different human stem cell from different origins (including pluripotent hESCS) even with low multiplicity of infection (MOI). Overall, the present invention offers an improved method to express and regulate the expression of a transgene in different types of human stem cells including pluripotent stem cells.

### BRIEF DESCRIPTION OF THE FIGURES

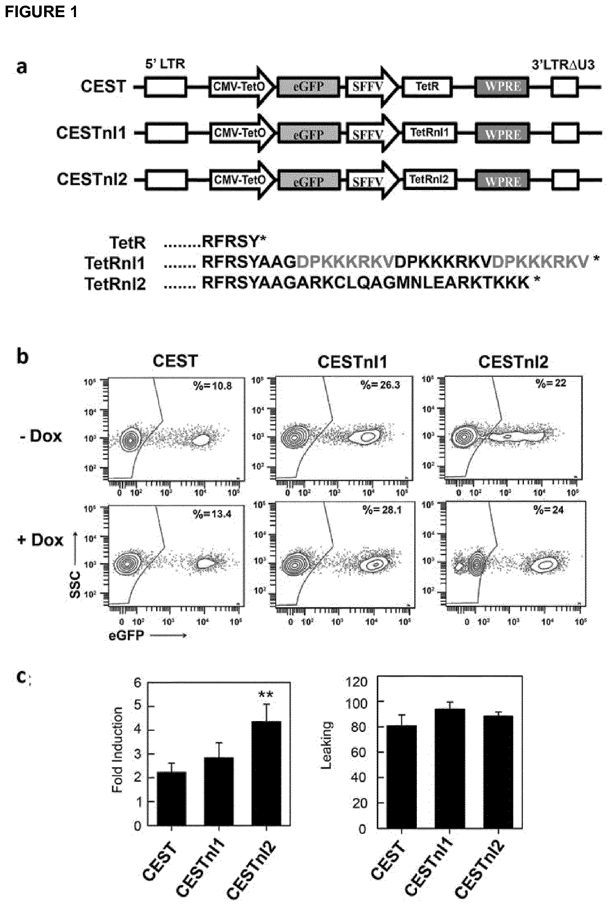

**Figure 1. Figure 1** **shows that the introduction of nuclear localization signals increase the fold induction of the CEST LV. (a)** Schematic representation of CEST, CESTnl1 and CESTnI2 lentiviral vectors (LVs) (top) and the NH3 terminus of the TetR, TetRnl1 and TetRnI2 (bottom). The TetRnl1 contain a linker peptide (AAG) and three tandem repeats of the nuclear localization signal of the SV40 T antigen (DPKKKRKV). In the TetRnl2 we included the same linker peptide and the nuclear localization signal of the glucocorticoid receptor (ARKCLQAGMNLEARKTKKK). (b) Representative plots showing eGFP expression of 293T transduced with different construct (CEST, CESTnI1, CESTnI2) in the absence (top) or presence (bottom) of Doxycycline (Dox). (c) Graphs showing fold induction (left) and leaking (right) of the different LVs in 293 T. Values represent mean +/- standard error of the mean of at least three separate experiments (**p<0.01)

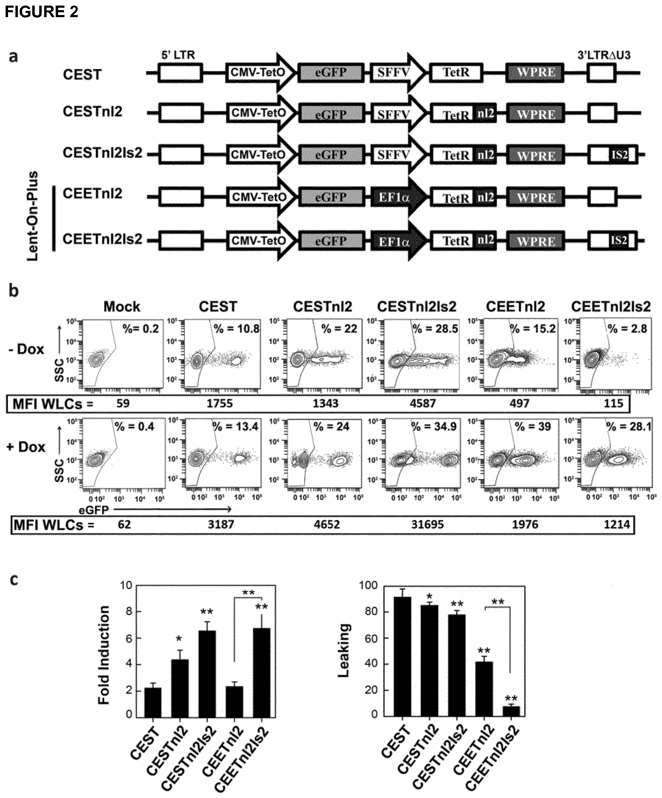

**Figure 2. Figure 2** **shows that the second-generation all-in-one LVs achieves tight transgene regulation in 293T cells with just one copy integration/cell.** (a)Schematic representation of the CEST LV²² (top) and the new all-in-one Tet-On LVs developed in this study (CESTnI2, CESTnI2Is2 and the Lent-On-Plus LVs: CEETnI2 and CEETnI2Is2). CMV (CMV-TetO); eGFP (enhanced green florescence protein); SFFV (spleen focus forming virus LTR promoter); hEF1α (human eukaryotic translocation elongation factor α1 promoter); TetRnl2 (TetR repressor incorporating the nuclear localization signal of the glucocorticoid receptor: see Fig. S1 for details). **(b)** Representative plots showing eGFP expression profiles of untransduced 293T (Mock) and 293T cells transduced with the different LVs (as indicated at the top of each plot) in the absence (top) or presence (bottom) of Dox (0.1µg/ml). A MOI = 0.3 was used to keep the percentage of eGFP⁺ cells below 40% (in order to keep transduced cells with only one LV integration). The gates of the eGFP⁺ populations were set to 0.2-0.4% of eGFP⁺ cells in the untransduced population (Mock; left plots) and subtracted to the % obtained under the different vectors and conditions for the analysis. The percentage (%) of the eGFP⁺ population (used to measure leaking) are shown in each plot. The MFI of the whole living cells (WLC) (used to measure fold induction) are indicated at the bottom of each plot **(c).** Graphs showing fold induction (left) and leaking (right) of the different LVs in 293T. Fold induction and leaking were calculated as indicated in M&M. To measure leaking, the backgrounds (% of eGFP⁺ of Mock cells) were subtracted to the % of the eGFP⁺ under the different conditions. Values represent mean +/- standard error of the mean of at least four separate experiments (* p<0.05; ** p<0.01). Asterisks indicate significance related to CEST (on top of the bars) or significance between the CEETnl2 and CEETnI2Is2 (as indicated in the Figure)

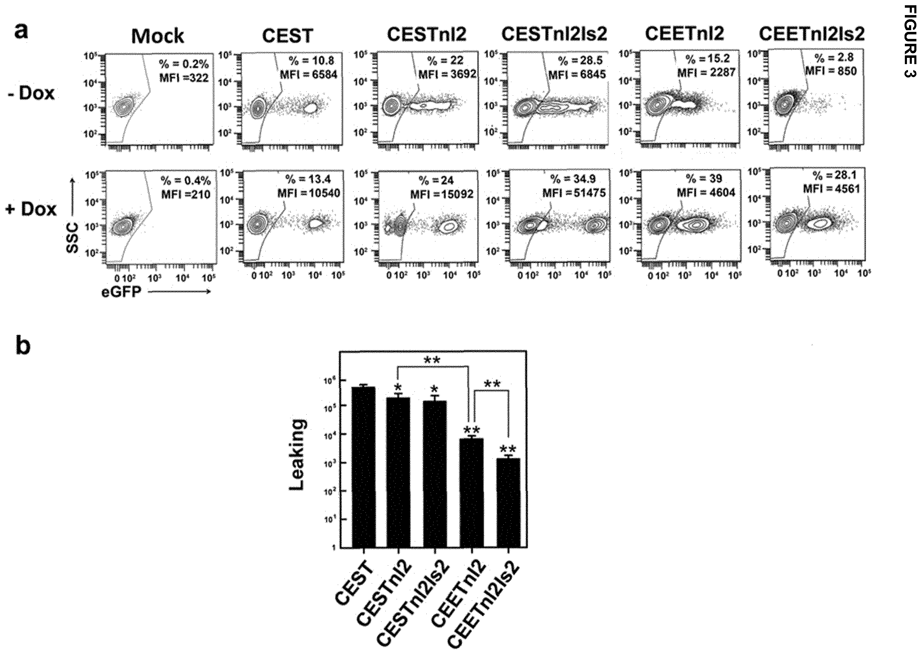

**Figure 3****. Figure shows the leaking of the different LVs in 293T using an alternative analysis.**
   **(a)** Representative plots showing eGFP expression profiles of untransduced 293T (Mock) and 293T cells transduced with the different LVs (as indicated at the top of each plot) in the absence (top) or presence (bottom) of Dox (0.1µg/ml). A MOI = 0.3 was used to keep the percentage of eGFP+ cells below 30% (in order to keep transduced cells with only one LV integration). The gates of the eGFP+ populations were set to 0.2-0.4% of eGFP+ cells in the untransduced population (Mock; left plots) and subtracted to the % obtained under the different vectors and conditions for the analysis. The percentage (%) of the eGFP+ population (used to measure leaking) and the Mean Fluorescence Intensity (MFI) of the eGFP+ population are shown in each plot **(b).** Graph showing leaking of the different LVs in 293T taking into account the MFI of the eGFP+ population in the absence of Dox: (Leaking = [[%eGFP+(-Dox) *100/%eGFP+(+Dox)] * MFI eGFP+ (-Dox)]. To measure leaking, the background (% of eGFP+ of Mock cells) were subtracted to the % of the eGFP+ under the different conditions. Values represent mean +/standard error of the mean of at least four separate experiments Asterisks indicate significance related to CEST (on top of the bars) or significance between the CEETnl2 and CEETnl2ls2 (as indicated in the Figure)(* p<0.05; ** p<0.01).

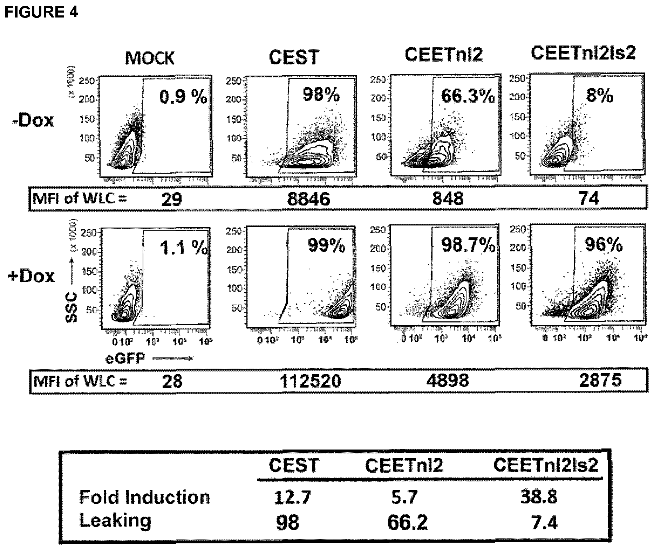

**Figure 4. Figure 4** **shows that the CEETnI2Is2 LV achieves high fold induction and low** l**eaking of on 293T transduced at high MOI**.Top: plots showing eGFP expression of untransduced (Mock) and CEST- CEETnI2- and CEETnI2Is2- transduced 293T cells in the absence (top) or presence (bottom) of Doxycycline (Dox). The MFI of whole living cells (WLC) are shown at the bottom of each plot. Fold induction and leaking of the different transduced populations are indicated in the table (bottom).

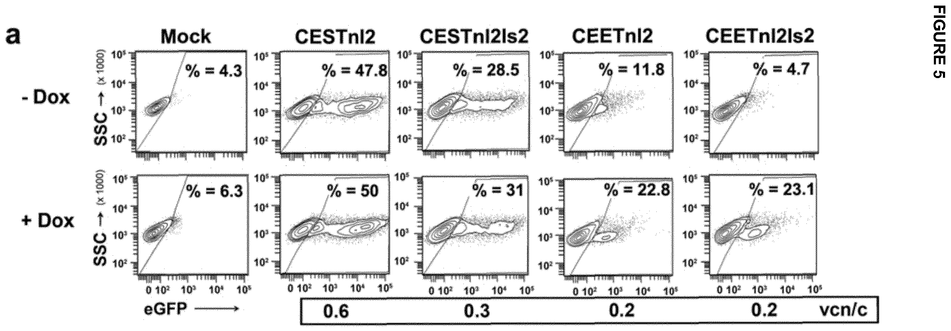

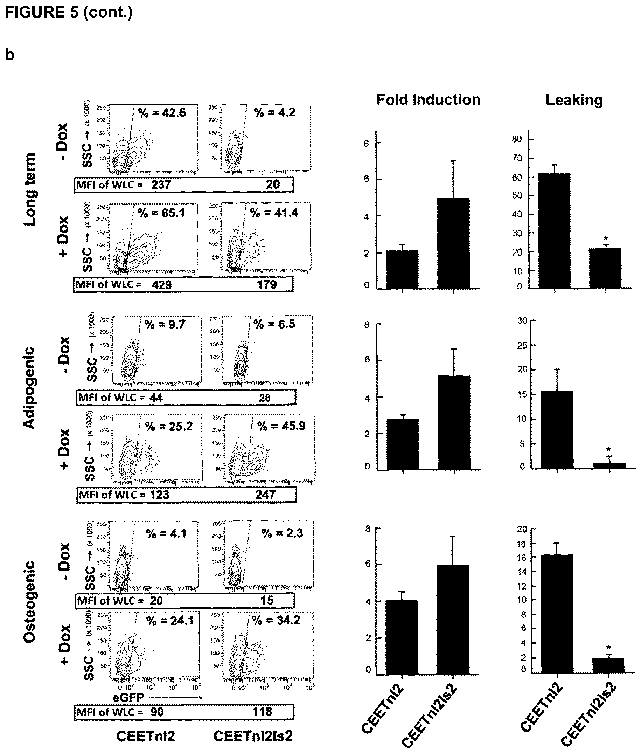

**Figure 5. Figure 5** **shows the performance of the different Tet-On LVs on hMSCs. (a)**Representative plots showing eGFP expression profiles of untransduced hMSCs (Mock) and hMSCs transduced with CESTnI2, CESTnI2Is2, CEETnl2 and CEETnl2ls2 LVs in the absence (top) or presence (bottom) of Dox (0.1µg/ml). Since hMSCs are more resistant to LVs transduction than 293T cells, a MOI = 1 was used to keep the percentage of transduced hMSCs below 40%. Vector copy number per cell (vcn/c) is indicated at the bottom of each plot. The gates of the eGFP⁺ populations were set to have 4-6% of eGFP⁺ cells in the untransduced population. The percentage (%) of the eGFP⁺ population are shown in each plot **(b)** hMSCs transduced with the Lent-On-Plus (CEETnI2 and CEETnI2Is2) LVs where kept in culture for up to 40 days (top plots and graphs) or differentiated toward the adipogenic (middle plots and graphs) and osteogenic (bottom plots and graphs) lineages. Representative plots showing eGFP expression profiles in the absence or presence of Dox are shown at the left and graphs showing fold induction and leaking are shown at the right. The MFI of the whole living cells (WLC) (used to measure fold induction) are indicated at the bottom of each plot. The gates of the eGFP⁺ were set to have 0.6%, 5,9% and 1.7% of eGFP⁺ cells in the untransduced population of the long term, adipogenic and osteogenic cells respectively (Fig. S5). Values represent mean +/- standard error of the mean of at least three separate experiments (* p<0.05)

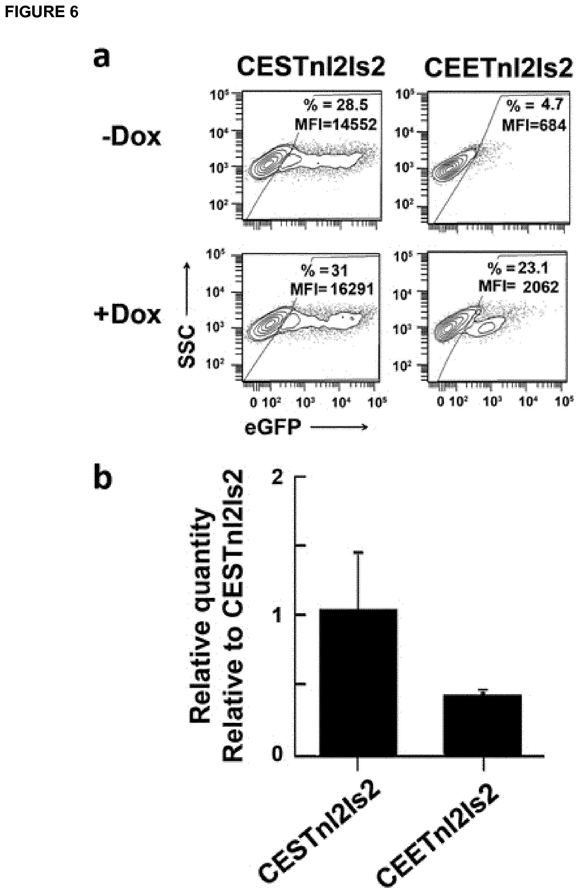

**Figure 6. Figure 6** **shows the analysis of the TetR expression levels of the different LVs backbones. (a)** Regulation of CESTLN2IS2 and CEETLN2!ns2 in human Mesenchymal Stem Cells (hMSCs). Representative plots showing eGFP expression profiles of hMSCs transduced with CESTnl2ls2 and CEETnl2ls2 LVs in the absence (top) or presence (bottom) of Dox. The percentage (%) and mean fluorescence intensity (MFI) of the eGFP+ population are shown in each plot. **(b)** Relative expression of the TetR mRNA CESTnI2- and CEETnI2-transduced hMSCs. TetR gene expression was assessed by RT-qPCR (see M&M for details). Samples were normalized using the housekeeping gene Gadph. Relative expression levels were calculated by the 2-ΔΔCT method normalized to the CESTnl2 data. Data are means +/standard error

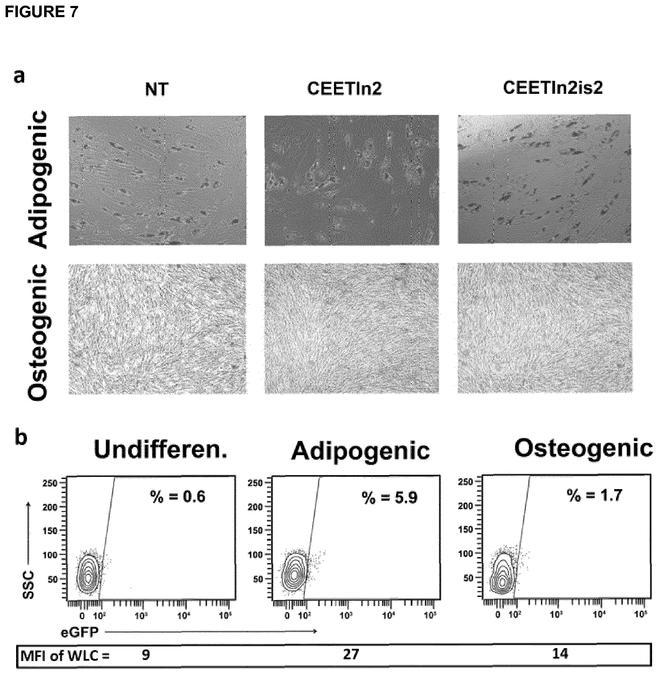

**Figure 7. Figure 7** **shows the analysis of multipotency of transduced hMSCs and the analysis of autofluorescence of the untransduced populations. (a)**Untransduced (Mock), CEETnI2- and CEETnI2Is2- transduced hMSCs were differentiated toward the adipoogenic (top panels) or osteogenic (bottom panels) and stained with oil red and Alizarin Red S respectively (See supplementary Methods for details). **(b)** Representative plots of the background fluorescence of the undifferentiated MSCs (left), adipogenic (middle) and osteogenic (reight) cells. The MFI of whole living cells (WLC) are shown at the bottom of each plot.

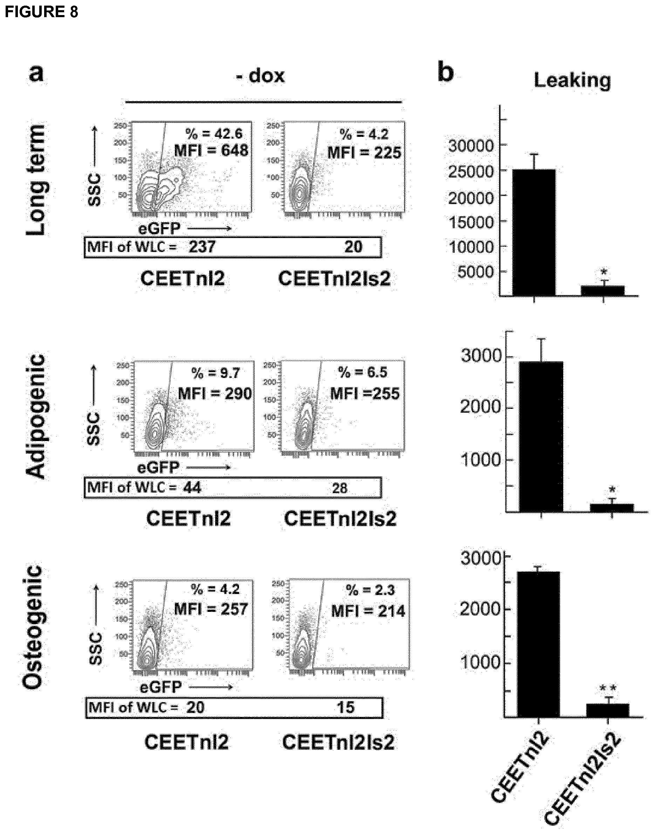

**Figure 8. Figure 8** **shows the kleaking of the CEETnl2 and CEETnI2Is2 in MSCs using an alternative analysis. (a)** Representative plots showing eGFP expression profiles of hMSCs transduced with CEETnl2 and CEETnl2ls2 LVs in the absence of Dox after 40 days in culture (long term) and after differentiation toward the adipogenic (middle plot) and osteogenic (bottom plots) lineages. The gates of the eGFP+ were set to have 0.6%, 5,9% and 1.7% of eGFP+ cells in the untransduced population of the long term, adipogenic and osteogenic cells respectively (Fig. S5). The percentage (%) and the MFI of the eGFP+ population are shown in each plot **(b)** Graphs showing leaking taking into account the MFI of the eGFP+ population in the absence of Dox: (Leaking = [[%eGFP+(-Dox) *100/%eGFP+(+Dox)] * MFI eGFP+ (-Dox)]. To measure leaking, the background (% of eGFP+ of Mock cells) were subtracted to the % of the eGFP+ under the different conditions. Values represent mean +/- standard error of the mean of at least three separate experiments (* p<0.05; ** p<0.01)

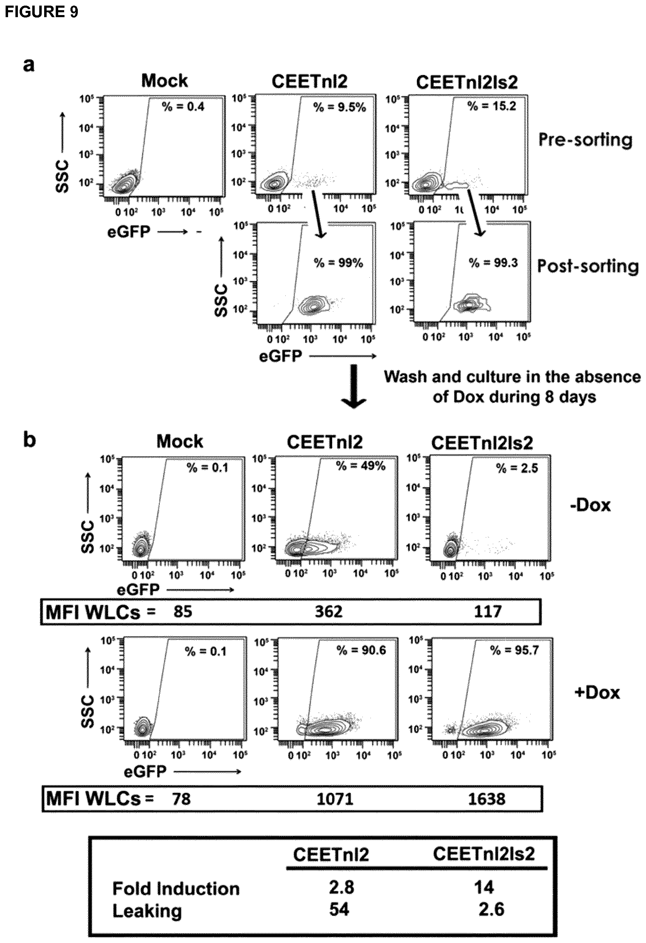

**Figure 9. Figure 9** **shows the performance of the Lent-On-Plus LVs on sorted hMSCs.** (a)Plots showing eGFP expression before (top plots) and after (bottom plots) purification of the eGFP⁺ populations of CEETnl2 and CEETnI2Is2-transduced hMSCs using fluorescenceactivated cell sorting (FACS) Aria II Flow Cytometer. A MOI = 0.4 was used to keep the percentage of transduced hMSCs below 15%. **(b)** After sorting, the hMSCs were kept in the absence of Dox for 8 days and then induced (+Dox) or not (-Dox) for additional 5 days and analyzed for eGFP expression. The Percentages (%) of eGFP⁺ cells in the different population are shown inside each plot. The MFI of the whole living cells (WLC) (used to measure fold induction) are indicated at the bottom of each plot. Fold induction and leaking of the CEETnI2and CEETnI2Is2-transduced sorted populations are indicated in the table (bottom).

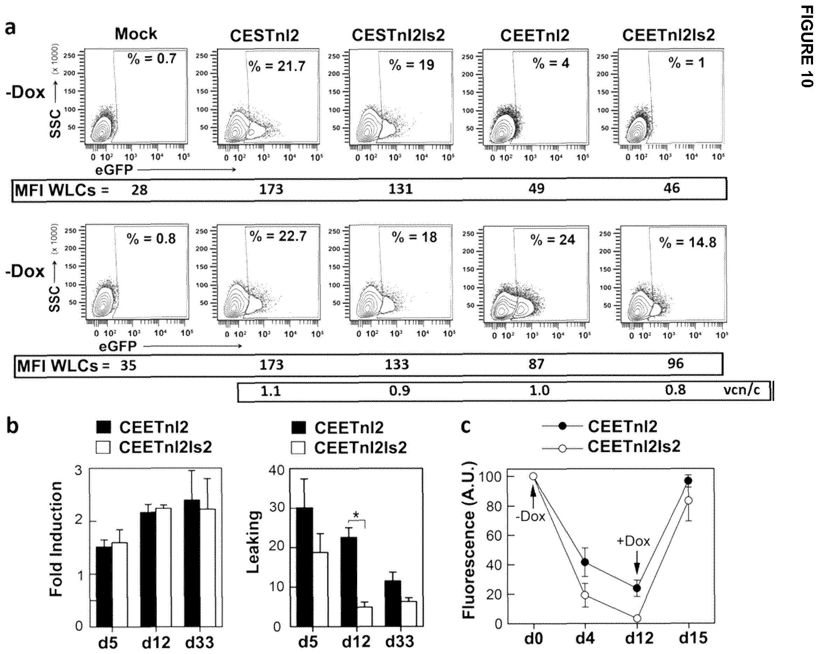

**Figure 10****. Figure shows that the Lent-On-Plus (CEETIn2 and CEETIn2Is2) LVs efficiently generate Doxycycline-responsive hESCs without selection or cloning.(a)**Representative plots showing eGFP expression profiles of hESCs (AND-1) control (Mock) and hESCs transduced with CESTnl2, CESTnl2ls2, CEETnl2 and CEETnl2ls2 LVs in the absence (top) or presence (bottom) of Dox (0.1µg/ml). hESCs were transduced at MOI=5 with the different LVs and analyzed 10 days later. The percentages (%) of the eGFP⁺ populations are shown in each plot. The MFIs of the whole living cells (WLC) are indicated at the bottom of each plot. The vcn/c of transduced cells are shown at the bottom of the figure. **(b)**Graphs showing fold induction (left) and leaking (right) of the CEETnl2 and CEETnl2ls2 LVs in hESCs at different time points during expansion (day 5, day 12 and day 33 post-transduction). Values represent mean +/- standard error of the mean of at least three separate experiments using AND-1 and H9 hESCs transduced at MOI=5 with the different LVs (* p<0.05). (c)Graph showing relative eGFP expression levels of CEETnl2 and CEETnI2Is2-transduced hESCs (AND-1) upon withdrawal (day 0) and addition (Day 12) of Dox along time in culture. Data represent fluorescence intensity relative to Day 0. Values represent mean +/- standard error of the mean of at least three separate experiments.

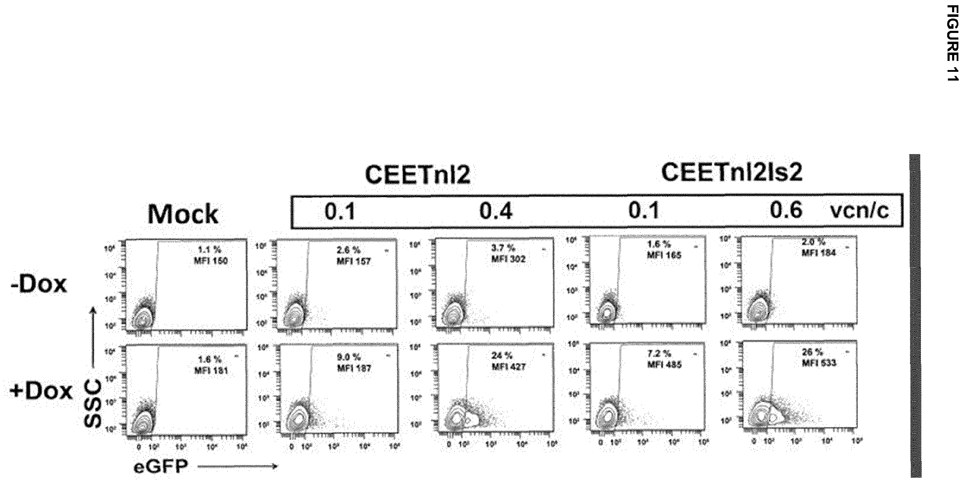

**Figure 11. Figure 11** **shows that the LentOnPlus LVs regulate transgene expression in hESCs at low vcn/c.** Representative plots showing eGFP expression profiles of hESCs (AND-1) transduced with CEETnl2 and CEETnl2ls2 LVs in the absence (top) or presence (bottom) of Dox. hESCs were transduced at two different MOls for each vector (MOI=0.3 and MOI = 1) in order to achieve a low transduction level. The percentage (%) and mean fluorescence intensity (MFI) of the eGFP+ population were analyzed by flow cytometry.

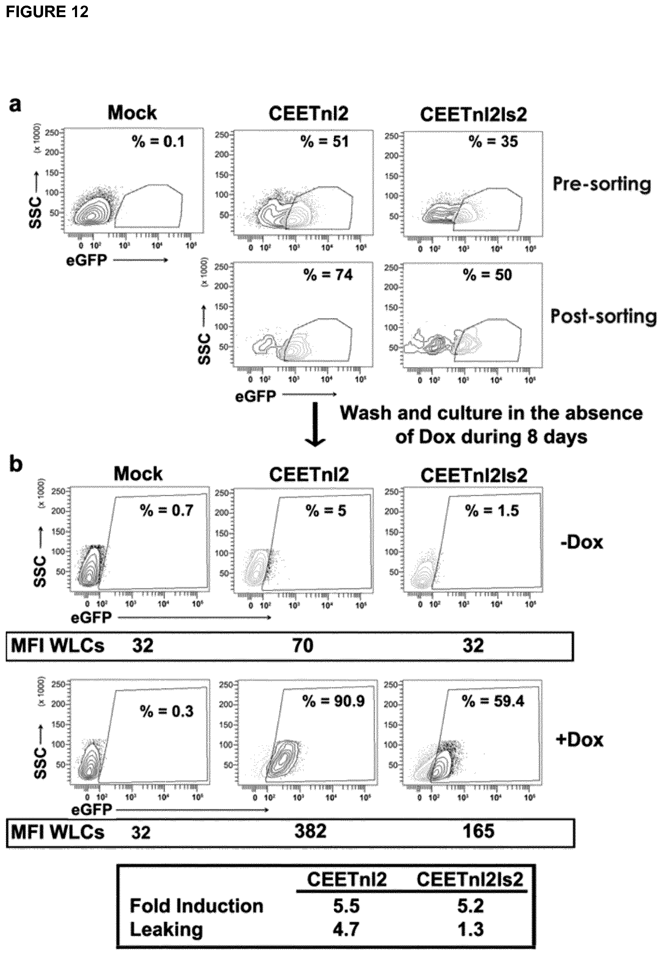

**Figure 12. Figure 12** **shows the performance of the Lent-On-Plus LVs on sorted hESCs. (a)**Plots showing eGFP expression before (Pre-sorting) and after (Post-sorting) purification of the eGFP⁺ populations of CEETnl2 and CEETnI2Is2-transduced hESCs using fluorescenceactivated cell sorting (FACS) Aria II Flow Cytometer. A MOI = 30 was used to achieve over 35% eGFP⁺ cells and good expression levels. **(b)** Sorted hESCs were kept in the absence of Dox for 8 days and then induced (+Dox) or not (-Dox) for additional 5 days and analyzed for eGFP expression. The Percentages (%) of eGFP⁺ cells are shown inside each plot. The MFI of the whole living cells (WLC) (used to measure fold induction) are indicated at the bottom of each plot. Fold induction and leaking of the CEETnI2- and CEETnI2Is2-transduced sorted populations are indicated in the table (bottom).

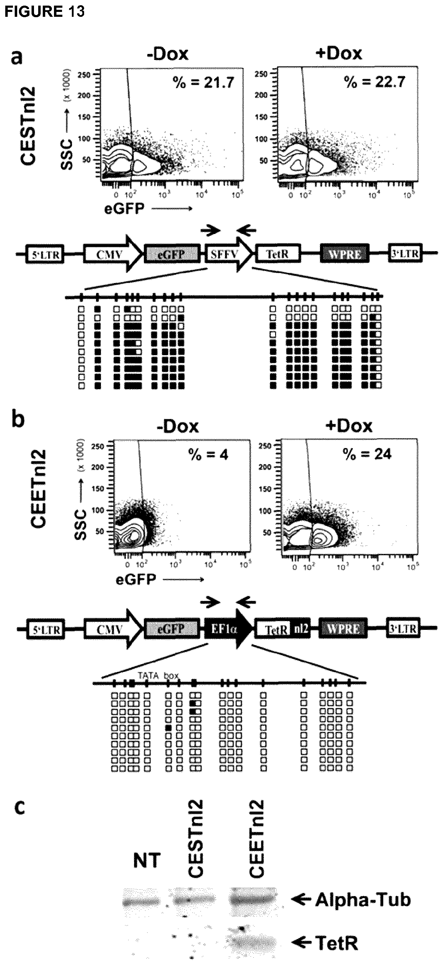

**Figure 13. Figure 13** **shows the methylation analysis of all-in-one Tet-ON LVs in hESCs.DNA** from CESTnI2- (a) and CEETnI2- (b) transduced-hESCs were extracted at day 15 post-transduction and converted with sodium bisulfate (see M&M for details). Plots showing Dox responsiveness of CESTnl2 and CEETnl2 at the time of lysis are shown at the top of (a) and (b) respectively. The converted DNA was subjected to PCR using the primers pair indicated in each figure and sequenced. The drawing below the LV schemes (bottom panels in (a) and (b)) represents the CpG islands contained in the promoter region that was analyzed. Black and white boxes represent methylated and unmethylated CpG respectively. It can be observed that SFFV is highly methylated in AND-1 (a) while hEF1α is highly resistant to methylation (b). (c)TetR expression in CESTnI2- and CEETnl2-transduced hESCs. hESCs (AND-1) transduced with the CESTnl2 and CEETnl2 were lysed and analyzed for TetR expression by Western Blot (see M&M for details)

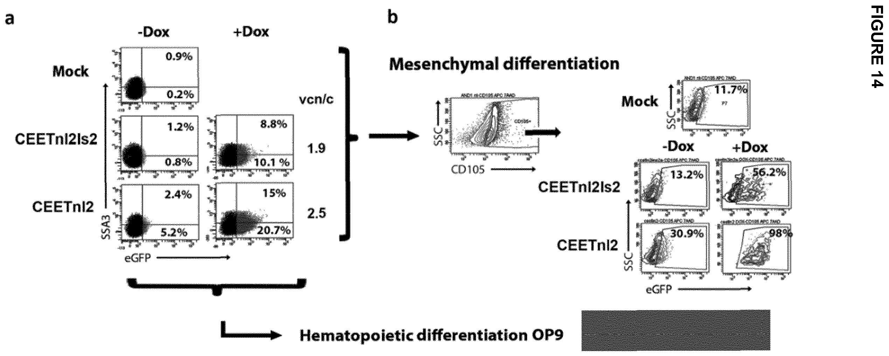

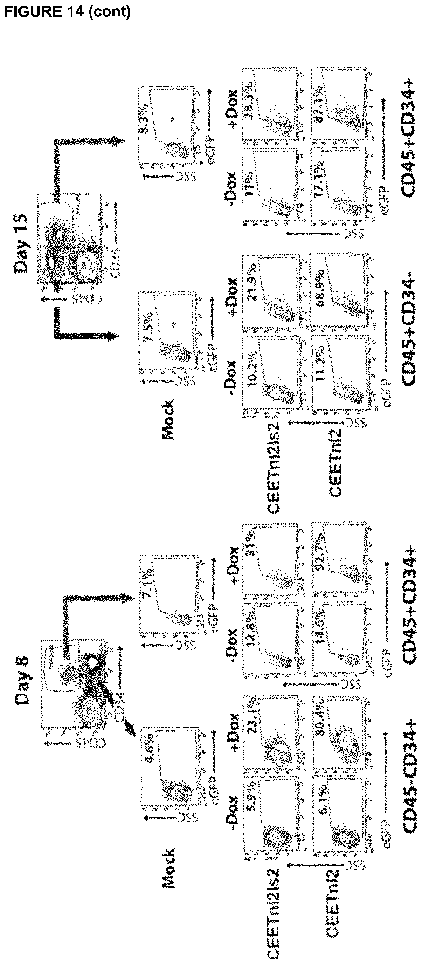

**Figure 14. Figure 14** **shows the transgene regulation of Lent-On-Plus (CEETnl2 and CEETnI2Is2) in mesenchymal and hematopoietic cells derived from hESCs. (a)** AND-1 hESC were transduced with concentrated Lent-On-Plus (CEETnl2 and CEETnl2ls2) LVs. eGFP and SSE3 (a pluripotency marker) expressions were analyzed in the presence (left plots) or absence (right plots) of Dox (0.1µg/ml). The gate of the eGFP⁺ populations were set to have 0.5-1-% of SSE3⁺eGFP⁺ cells in untransduced population (Mock; top plot). **(b)** Lent-On-Plus regulatable hESCs were differentiated to mesenchymal stromal cells (see M&M for details). At day 10 of differentiation Dox was added and eGFP expression levels analyzed 5 days later. The plots show eGFP expression levels in the CD105 population of untransduced (Mock; top plot), CEETnI2Is2-transduced (middle plots) and CEETnl2 (bottom plots) in the presence or absence of Dox as indicated at the top of the plots. (c)Lent-On-Plus regulatable hESCs were differentiated toward the hematopoietic lineage using the OP9 differentiation protocol (see M&M for details). The cells were maintained in the presence or absence of Dox from day 1 of differentiation and the eGFP expression analyzed in the different hematopoietic populations (CD45⁻CD34⁺, CD45⁺CD34⁺ and CD45⁺CD34⁻) as indicated at the different days (Day 8; Top-Left plot and Day 15; Top-right plot). Background levels in untransduced hESCs were set between 4.6%-8.3% for each population (arrows) at each day of differentiation (Mock, second top plots). The percentage of cells expressing eGFP in the presence or absence of Dox of the CEETnI2- and CEETnI2Is- transduced hESCs are shown inside each plot for each hematopoietic population.

### DESCRIPTION

### Definitions

- In the context of the present invention, coding nucleic acid molecules are understood to be DNA sequences that, when transcribed into RNA molecules through the action of the regulatory elements, will give rise to functional proteins or RNAs.
- In the context of the present invention, "genetic construct" is understood as a constructed segment of nucleic acid that is going to be "introduced" into a target tissue or cell. It consists on a vector that may contain a gene cassette, which comprises the nucleic acid sequence encoding at least a protein of interest and different regulatory sequences such as promoters or enhancers for expression in the host organism. It can be simple or double stranded. Optionally, the genetic construct is made of, or comprises, modified nucleic acid bases or morpholinos. Generally, genetic constructs express a wild-type protein, express mutant proteins that may contain deletion mutations or missense mutations, or prevent the expression of certain genes, by expressing inhibitor or competitor proteins, or by encoding silencing RNAs (i.e, siRNAs, miRNAs , piRNAs, snRNAs, etc) or morpholinos.
- In the context of the present invention, the term "vector" is referred to a small piece of DNA, taken from a virus, a plasmid (commonly found in bacteria), or the cell of a higher organism into which foreign genetic fragment/s can be inserted and is commonly used as a vehicle to artificially carry this foreign genetic material into another cell, where it can be replicated, expressed, and/or inserted into the host genome. The vector itself is generally a DNA sequence that consists of (a) transgene/s and a larger sequence that serves as the "backbone" of the vector. It can be simple or double stranded. Optionally, it can be formed or comprise modified nucleic acid bases or morpholinos.
- In the context of the present invention, the term "gene cassette" refers to a natural or recombinant nucleic acid sequence capable of expressing at least one nucleic acid molecule. This expression might give rise to an RNA that may be, or not, translated into a protein. It may optionally be formed or comprise modified nucleic acid bases or morpholinos. It typically comprises at least one nucleic acid molecule to be expressed and at least one promoter (allowing transcription initiation). Optionally, the gene cassette may include additional nucleic acid molecules to be expressed, additional regulatory sequences, such as transcriptional enhancers or transcription termination signals, and non-coding sequences, splicing signals, translation termination signals, polyadenylation signals, polyadenosine sequences, or internal ribosome entry sites (IRES). In the present invention the gene cassette is the nucleic acid sequence of the genetic construct that is aimed to be introduced into the genome of the target cell that then becomes the host cell.
- In the context of the present invention, the term "transgene" refers to the nucleic acid molecule of the gene cassette of the genetic construct that encodes the protein or RNA molecule whose expression and/or expression regulation in the host genome is the ultimate goal of the present invention. It is the coding nucleic acid molecule whose expression is regulated by the protein encoded by the other nucleic acid molecule present in the gene cassette. It may optionally be formed or comprise modified nucleic acid bases or morpholinos.
- In the context of the present invention, "gene expression" is understood as the process by which the nucleotide sequence of a gene is used to direct transcription and ultimately protein sytnhesis. The process of gene expression involves two main steps:
   1) Transcription: the production of messenger RNA (mRNA) by an RNA polymerase using the DNA sequence as "template". This step is followed by the processing or maturation of the resulting mRNA molecule.
   2) Translation: the use of mRNA to direct protein synthesis. This second step can be followed by post-translational modifications of the protein molecule.

Additionally, in some cases gene expression leads to the production of other forms of RNA that are not translated into proteins, such as for example transfer RNAs (tRNAs), ribosomal RNAs (rRNAs), long non-coding (IncRNAs), or RNAs that will derive into micro-RNAs (miRNAs) or small interfering RNAs (siRNAs).
- In the context of the present invention, an insulator element is understood to be a DNA sequence that isolates genes located in one chromatin domain from the effect of enhancers or silencers present in neighboring domains and vice versa. The insulator element of the present invention is capable of blocking the regulatory action of the regulatory elements placed within its range of action and permits the expected expression of coding nucleic acid molecules (please note that it is herein understood that the term "coding nucleic acid molecules" includes the term "transgene") in research, protein production and gene therapy in mammalian cells, preferably in embryonic and adult stem cells.
- In the context of the present invention, a nuclear localization signal or sequence refers an amino acid sequence that facilitates the import into the cell nucleus of the proteins it is physically associated with by protein-protein interaction or because it forms part of the said protein.
- In the context of the present invention, regulatory control elements are understood to be DNA sequences able to promote the synthesis of RNAs under specific conditions.
- In the context of the present invention, a mammalian cell refers to cells which are derived or isolated from tissue of a mammal.
- In the context of the present invention, the term "multiplicity of infection (MOI)" refers to the ratio of the number of genetic constructs containing the gene cassette to be introduced in the target cells, to the number of target cells in a defined space.

### Description

The present invention offers a genetic construct capable to introduce a specific gene cassette into the genome of a target cell, that promotes a better regulation of a transgene and that requires a lower MOI, compared to previous constructs of the same type. Additionally, this construct allows for significant expression of the transgene in different human stem cells where previous constructs show no regulation of expression. Therefore, the present invention offers a solution to the need of technical improvements to reach tightly regulated transgene expression in bulk populations after one single round of transduction/transfection, especially for transgenes to be introduced in human stem cells.

The present invention refers to a genetic construct having nucleic acid sequences capable of integrating into the genome of a mammalian cell comprising:
a) two regulatory control elements, b) at least two coding nucleic acid molecules referred to as the first coding nucleic acid molecule and the second nucleic acid molecule, or as NAm-A and NAm-B respectively, operatively associated with the regulatory control elements and capable of expression in the cell, c) a nucleic acid molecule encoding a nuclear localization element, and d) an insulator element,
wherein NAm-B encodes a protein capable of repressing the regulatory control element, from now on referred as RCE-A, that controls the expression of NAm-A, wherein RCE-A is inducible and can be regulated by the protein encoded by NAm-B, wherein the nucleic acid molecule encoding the nuclear localization signal is associated to NAm-B so that both nucleic acid molecules encode a single polypeptide.

The nucleic acid molecule encoding the nuclear localization signal encodes the nuclear localization signal from the glucocorticoid receptor, referred to as nuclear localization signal 2 (nI2), preferably with nucleic acid sequence SEQ ID n°4 (gcccgaaaatgtcttcaggctggaatgaacctggaagctcgaaaaacaaagaag). The insulator preferably consists on the SEQ ID n°1, even more preferably consists on the SEQ ID n°1 associated with SEQ ID n°2 so that they can form a single nucleic acid sequence, yet more preferably consists on the SEQ ID n°3. In a preferred embodiment, the nucleic acid molecule encoding the nuclear localization signal is SEQ ID n°4 and the insulator the one with SEQ ID n°3.

In previous embodiments of the invention, suitable sequences preferably have at least 90% identity, at least 95% identity, at least 96% identity, at least 97% identity, at least 98% identity, at least 99% identity, at least 99.5% identity, over the total length of the sequence, to the sequence used to define them. Identity refers to the similarity of two nucleotide sequences that are aligned so that the highest order match is obtained. Identity is calculated according to methods known in the art. For example, if a nucleotide sequence (called "Sequence A") has 90% identity to a portion of SEQ ID NO 1, then Sequence A will be identical to the referenced portion of SEQ ID NO 1 except that Sequence A may include up to 10 point mutations (such as substitutions with other nucleotides) per each 100 nucleotides of the referenced portion of SEQ ID NO 1.

The invention also includes an insulator that has a nucleic acid sequence with sufficient identity to the insulator elements described previously in this section of the invention to hybridize with them under stringent hybridization conditions. In this sense, the present invention also includes insulator elements having nucleic acid molecules that hybridize to one or more of the sequences in SEQ ID n° I-SEQ ID n° 3 or its complementary sequence. Similarly, the invention also includes a nucleic acid sequence with sufficient identity to the nuclear localization signals described previously in this section to hybridize with them under stringent hybridization conditions. In this sense, the present invention also includes insulator elements having nucleic acid molecules that hybridize to SEQ ID n°4 its complementary sequence. Such nucleic acid molecules preferably hybridize under high stringency conditions (see Sambrook et al. Molecular Cloning: A Laboratory Manual, Most Recent Edition, Cold Spring Harbor Laboratory Press, Cold Spring Harbor, N.Y.). High stringency washes preferably have low salt contents (preferably about 0.2% SSC) and a temperature of about 50-65 °C. Thus, in another embodiment, the insulator element and/ or the nucleic acid molecule encoding the nuclear localization signal are introduced in the anti-sense orientation. Additionally, other insulator elements and/or nucleic acid molecules encoding other nuclear localization signals can be readily inserted in the genetic construct provided that expression of NAm-A and NAm-B still occurs and that the encoded nuclear localization signal promotes the import of NAm-B encoded protein into the nucleus of the host cell.

Commonly, the purpose of introducing a transgene in a host cell is to promote its expression in this cell, and ideally it should be possible to turn on and off its expression according to the needs. Since the protein encoded by NAm-B can activate or repress the expression of NAm-A, ideally this activator or repressor function on NAm-A expression might be regulatable according to the needs. A common mechanism of regulation is that NAm-B protein can be targeted by a molecule in the cell, that is itself present in the cell according to the needs. Therefore, in a preferred embodiment, NAm-B encodes a protein whose regulatory function is drug-responsive, preferably tetracycline - or a variant thereof - responsive. In a more preferred embodiment, the regulatory element is based on the original TetR repressor element. In the context of the present invention, it is understood that the original TetR repressor is the product of the TetR gene (the TetR protein) from *Escherichia coli.*

In order to regulate the expression of NAm-A, NAm-B encoded protein has to be able to bind to RCE-A that controls the expression of NAm-A. Thus, in another embodiment, the RCE-A comprises a binding site for the protein encoded by NAm-B. In addition, preferably the regulation of NAm-A expression by NAm-B encoded protein is as follows: in the absence of the drug described in the previous paragraph, binding of NAm-B encoded protein to RCE-A leads to the repression of RCE-A and to the inhibition of NAm-A expression.Thus in another preferred embodiment, NAm-B encodes a protein that binds to REC-A to inhibit the expression of NAm-A. Preferably, in the presence of the drug, Nam-B encoded protein cannot further repress the expression of NAm-A. Then, in a yet more preferred embodiment, in the presence of the drug described previously in this section, NAm-B cannot efficiently repress the expression of NAm-A. Yet, in another more preferred embodiment, RCE-A comprises the Cytomegalovirus (CMV) promoter.

To allow that the molecule that regulates the function of the NAm-B encoded protein (such as a tetracycline-derived drug) has an effect on NAm-A expression at any time, the expression of NAm-B might be constitutive. The regulatory element that controls the expression of NAm-B (from now on referred as RCE-B) is a constitutive promoter. Preferably, RCE-B is the spleen focus forming virus (SFFV) promoter. According to figure 2C and 10A, when RCE-B is the human elongation factor-1 α (hEF1α), basal expression of NAm-A in the absence of doxycycline (a tetracycline analogue) is significantly reduced, both in 293T cells and in human embryonic stem cells (hESCs), therefore the leaking is lower than when using the SFFV promoter as the RCE-B. Additionally, as shown in figure 13, methylation of hEF1α is much lower than that of SFFV, leading to a higher protein levels of the NAm-B encoded protein (Fig 13c). Thus RCE-B is the hEF1α promoter.

Overall, in a more preferred embodiment, the genetic construct of the first aspect of the invention comprises: an RCE-A that contains a binding site for the regulatory protein encoded by NAm-B and preferably further comprises the CMV promoter; an RCE-B that is constitutively active, is the hEF1α promoter; a NAm-B that encodes a protein whose function is drug-responsive, preferably tetracycline- or a variant thereof- responsive, more preferably is based on the TetR repressor element, yet more preferably it represses the expression of NAm-A in the absence of the drug; a nucleic acid molecule (associated with the NAm-B so that both nucleic acid molecules encode a single polypeptide) that encodes a nuclear localization signal, preferably encoding that of the glucocorticoid receptor referred as nI2, more preferably sharing at least 90% of identity over the total length of the sequence with the sequence ID SEQ n° 4, yet more preferably it is the sequence ID SEQ n°4; wherein the insulator sequence is the one sharing at least 90 % of identity over the total length of the sequence preferably with the one consisting on the SEQ ID n°1, even more preferably with the one consisting on the SEQ ID n°1 associated with SEQ n°2 so that they form a single nucleic acid molecule, yet more preferably with the one consisting on the SEQ ID n°3; more preferably, the insulator sequence is any of the ones just mentioned, more preferably it is SEQ ID n°3.

The final goal of gene transfer is to insert a gene into a cell (and ultimately into an organism) using a vector that will transfer *only* the required nucleic acid sequences into the desired target cells. Viruses are naturally very efficient at transducing their own genetic information into host cells for their own replication. By replacing non-essential viral genes with foreign genes of therapeutic interest, recombinant viral vectors can be used to transduce the cell type that they would normally infect. For biosafety and viral safety reasons, in addition to the transgene(s) of interest, these modified vectors in many cases contain/express only the viral sequences required for the initiation of viral DNA replication and packaging (Bouard D. et al., Viral vectors: from virology to transgene expression, British Journal of Pharmacology (2009)). Therefore, in a particular embodiment, the genetic construct according to any of the previous embodiments is a viral vector. Examples of viral vectors that are commonly used for this purpose are retrovirus vectors, lentivirus vectors, Adenovirus Vectors, Adeno-associated virus (AAV) vectors and Semliki Forest Virus.

Retroviruses are viruses that carry RNA as genetic material instead of DNA and usually also carry reverse transcriptase enzymes that convert RNA into its DNA copy. Then, this DNA is integrated into the host genome using integrase enzyme. Thus, retroviruses are a subtype of virus that integrate their own genetic information into the host cell genome. They are broadly used in different gene transfer techniques.

Retrovirus vectors contain 5' and 3' Long Terminal Repeats (LTRs) that are found at either end of proviral DNA formed by reverse transcription of retroviral RNA. They are used by retroviruses to insert their genetic material into the host genomes. Each LTR consists of a U3, R, and U5 region. The ends of the LTRs subsequently participate in integration of the provirus DNA into the host genome. Once the provirus has been integrated, the LTR on the 5' end serves as the promoter for the entire retroviral genome, while the LTR at the 3' end provides for nascent viral RNA polyadenylation. The 3' LTR usually acts in transcription termination and polyadenylation. For safety reasons, retroviral vectors have the LTR promoter (U3) deleted and use an internal promoter to express transgenes in transduced cells..

Lentiviruses are a subtype of retroviruses. They present several advantages compared to other retroviruses for gene transfer purposes: (i) broad tissue tropisms, including important gene- and cell-therapy-target cell types; (ii) contrary to most retroviruses, the capability of infecting both dividing and non-dividing cells; (iii) no expression of viral proteins after vector transduction; (iv) the ability to deliver complex genetic elements, such as polycistronic or intron-containing sequences; (v) potentially safer integration site profile; and (vi) a relatively easy system for vector manipulation and production (Sakuma T. et al., Lentiviral vectors: basic to translational, Biochemical Journal (2012)). Therefore, they have been used to transfer genes into delicate cells such as stem cells or neurons. (Miyoshi H et al., Transduction of human CD34+ cells that mediate long-term engraftment of NOD/SCID mice by HIV vectors. Science (1999); Lois C et al., Germline transmission and tissue-specific expression of transgenes delivered by lentiviral vectors. Science (2002)). The genetic construct of the present invention is a lentivirus vector (LV).

A second aspect of the invention refers to a composition comprising the genetic construct according to any of the previous embodiments, wherein this composition can be a pharmaceutical composition (from here in after pharmaceutical composition of the invention). The pharmaceutical composition of this invention can be used to treat patients having diseases, disorders or abnormal physical states such as blood diseases or neural diseases (neurodegenerative). Blood diseases treatable by stem cell transplant include leukemias, myelodysplastic syndromes, stem cell disorders, myeloproliferative disorders, lymphoproliferative disorders phagocyte disorders, inherited metabolic disorders, histiocytic disorders, inherited erythrocyte abnormalities, inherited immune system disorders, inherited platelet abnormalities, plasma cell disorders, and malignancies. Stem cell nerve diseases to be treated by neural stem cell transplantation include diseases resulting in neural cell damage or loss, eg. paralysis, Parkinson's disease, Alzheimer's disease, ALS, multiple sclerosis. The pharmaceutical composition of the invention can be administered by ex vivo and in vivo methods such as electroporation, DNA microinjection, liposome DNA delivery. Derivatives or hybrids of these vectors may also be used. Dosages to be administered depend on patient needs, on the desired effect and on the chosen route of administration.

The pharmaceutical composition of the invention could include acceptable carriers or excipients.

It can be prepared by known methods for the preparation of pharmaceutically acceptable compositions which can be administered to patients, and such that an effective quantity of the genetic construct is combined in a mixture with a pharmaceutically acceptable vehicle. Suitable vehicles are described, for example in Remington's Pharmaceutical Sciences (Remington's Pharmaceutical Sciences, Mack Publishing Company, Easton, Pa., USA). On this basis, the pharmaceutical composition could include an active compound or substance, such as the genetic construct, in association with one or more pharmaceutically acceptable vehicles or diluents, and contained in buffered solutions with a suitable pH and isosmotic with the physiological fluids. The methods of combining the genetic construct with the vehicles or combining them with diluents is well known to those skilled in the art. The composition could include a targeting agent for the transport of the active compound to specified sites within the target cells. Thus, in a particular embodiment of the invention, the pharmaceutical composition optionally comprises a pharmaceutically acceptable vehicle.

In addition, the present disclosure not forming part of the invention also includes compositions and methods for providing a genetic construct as defined in any of the embodiments of the first aspect of the invention, to a subject such that expression of the nucleic acid molecules of the genetic construct (NAm-A and NAm-B) in the cells provides the biological activity of the polypeptide encoded by the coding nucleic acid molecules in those cells. The disclosure not forming part of the invention includes methods and compositions for providing a coding nucleic acid molecule NAm-A to the cells of an individual such that the expression regulation of NAm-A in the cells provides the biological activity or phenotype of the polypeptide encoded by NAm-A to the cell. The method also relates to a method for providing an individual having a disease, disorder or abnormal physical state with a biologically active polypeptide by administering a genetic construct of the present invention. The amount of polypeptide will vary with the subject's needs. The optimal dosage of genetic construct may be readily determined using empirical techniques, for example by escalating doses. Various approaches to gene therapy may be used. The disclosure not forming part of the invention includes a process for providing a human with a therapeutic polypeptide including: introducing human cells into a human, said human cells having been treated in vitro or ex vivo to insert therein a pharmaceutical composition of the invention or a genetic construct of the invention, the human cells expressing in said human a therapeutically effective amount of said therapeutic polypeptide.

The method also relates to a method for producing a stock of recombinant virus by producing virus suitable for gene therapy. This method preferably involves transfecting cells permissive for virus replication and collecting the virus produced.

Furthermore, the disclosure not forming part of the invention also relates to a mammalian host cell (isolated cell in vitro, a cell in vivo, or a cell treated ex vivo and returned to an in vivo site) comprising the genetic construct of any of the embodiments of the first aspect of the invention. In this sense, cells transfected with a nucleic acid molecule as a DNA molecule, or transduced with the nucleic acid molecule as a DNA virus vector, may be used, for example, in bone marrow or cord blood cell transplants according to techniques known in the art. Examples of the use of transduced bone marrow or cord blood cells in transplants are for ex vivo gene therapy of Adenosine deaminase (ADA) deficiency. Other cells which may be transfected or transduced either ex vivo or in vivo include purified stem cells. Thus, the disclosure not forming part of the invention refers to a mammalian host stem cell comprising the genetic construct according to any of the embodiments of the first aspect of the invention. In particular, the host stem cell is a cell of embryonic or adult tissue origin.

In any case such a mammalian cell or mammalian host cell transfected or transduced with the genetic constructs can be useful as research tools to measure levels of expression of the coding nucleic acid molecule NAm-A and the activity of the polypeptide encoded by NAm-A. In this sense the genetic constructs of the invention are useful in research to deliver marker genes or antisense RNA to cells. In particular, the disclosure not forming part of the invention refers to the use of the genetic construct according to any of the embodiments of the first aspect of the invention, for cell marking.ln particular, the disclosure not forming part of the invention refers to the use of the genetic construct according to any of the previous embodiments, for cell genetic manipulation studies.

A fourth aspect of the invention refers to an in vitro method for expressing a nucleic acid molecule in a mammalian host cell, comprising a) administering to the cell an effective amount of the genetic construct according to any of the previous embodiments, and b) expressing the nucleic acid molecules of the genetic construct to produce the coding nucleic acid molecule RNA and its encoding polypeptide. Preferably, the host cell is a stem cell.

A fifth aspect of the invention refers to an in vitro method for producing a polypeptide in a mammalian host cell, comprising a) administering to the cell an effective amount of the genetic construct according to any of the previous embodiments, and b) expressing the nucleic acid molecule of the genetic construct to produce the coding nucleic acid molecule RNA and its encoding polypeptide. Preferably, the host cell is a stem cell.

In a preferred embodiment of the fourth and fifth aspects of the invention, the host cell is a pluripotent stem cell of embryonic origen (ESCs) or adult tissue origin (iPS). In a particular embodiment, the host cell is a cell factory for protein production (ie CHOs, HEK free style).

The following examples are only meant for illustrative purposes.

### EXAMPLES

### Materials and methods

### Plasmids construction

Two different nuclear sequences named nl1 and nI2 were incorporated at the 3'end of TetR preceding the stop codon in the StetR LV (Benabdellah, K. et al., Development of an all-in one lentiviral vector system based on the original TetR for the easy generation of Tet-ON cell lines. PLoS ONE (2011)) to generate STetRnl1 and STetRnI2. nl1 consist in a three tandem repeat sequence corresponding to the nuclear localization signal (DPKKKRKV) from SV40, whereas nI2 (RKCLQAGMNLEARKTKK) is the nuclear localization signal from the glucocorticoid receptor. A Pstl-Pstl 1231 bp fragment from the STetRnl1 and STetRnI2 LVs was inserted within the unique Pstl site of the CEWP vector (Demaison, C. et al. High-level transduction and gene expression in hematopoietic repopulating cells using a human immunodeficiency [correction of imunodeficiency] virus type 1-based lentiviral vector containing an internal spleen focus forming virus promoter. Hum Gene Ther (2002)) to get the CESTnl1 and CESTnl2 respectively. CESTnl2ls2 were constructed by inserting a Kpnl-Nhel 1509bp fragment from the SE-ls2Rev (Benabdellah, K. et al., A chimeric HS4-SAR insulator (IS2) that prevents silencing and enhances expression of lentiviral vectors in pluripotent stem cells. PLoS ONE (2014)) into the Kpnl-Nhel site of CESTnI2. CEETnl2 and CEETnl2ls2 were constructed in three steps: 1- a DNA fragment encompassing the TetRnl2 sequence was amplified from the StetRnI2 and inserted into the pLVTHM LV (Addgene plasmid 12247) using standard molecular techniques to obtain the pLVTHM-TetRnI2. 2- The CEETnl2 vector was generated by insertion of a Sall-Kpnl fragment (encompassing the hEF1α-TetRnI2) into the Pstl site of CEWP. 3- Finally, the CEETnl2ls2 was constructed by inserting the Kpnl-Nhel 1509bp fragment from the SE-ls2Rev Benabdellah, K. et al., A chimeric HS4-SAR insulator (IS2) that prevents silencing and enhances expression of lentiviral vectors in pluripotent stem cells. PLoS ONE (2014) into the Kpnl-Nhel site of CEETnI2.

*Lentiviral vector production, cells transduction and calculation of vector copy number per cell.* The human immunodeficiency virus (HIV) packaging (pCMV_R8.91) and VSV-G (pMD2.G) plasmids (http://www.addgene.org/Didier_Trono) are described elsewhere (Zufferey, R. et al., Multiply attenuated lentiviral vector achieves efficient gene delivery in vivo. Nat.Biotechnol. (1997); Naldini, L. et al. In vivo gene delivery and stable transduction of nondividing cells by a lentiviral vector. Science (1996)). Vector production was performed as previously described (Benabdellah et al., 2011). Briefly, 293T cells were plated on amine-coated petri-dishes (Sarsted,Newton, NC). The vector (CEST, CESTnI1, CESTnl2, CESTnl2ls2, CEETnl2 or CEETnI2Is2), the packaging (pCMVΔR8.9) and the envelope (pMD2.G) plasmids were transfected into the 293T cells using LipoD293 (SignaGen, Gainthersburg, MD, USA). Viral supernantants were collected and frozen or concentrated by ultrafiltration at 2000 g and 4°C, using 100 Kd centrifugal filter devices (Amicon Ultra-15, Millipore, Billerica, MA). Freshly collected virus particles, were used to transduce 293T, hMSCs and hESC as previously described (Benabdellah, K. et al., Development of an all-in-one lentiviral vector system based on the original TetR for the easy generation of Tet-ON cell lines. PLoS ONE (2011); Munoz, P. et al. Specific marking of hESCs-derived hematopoietic lineage by WAS-promoter driven lentiviral vectors. PLoS ONE (2012)) Briefly, hMSCs were dissociated and mixed with the viral particles at the desired MOI, left at room temperature for 10 minutes, seeded and maintained at the incubator (5% O2; 5% CO2; 37° C and humidity) for 5 hours. hMSCs were then washed and seeded in a bigger flask for expansion. hESCs were dissociated with collagenase type IV and plated on fresh matrigel in the presence of the fresh viral particles. The media was changed after 5 hours. When the colonies reached confluence they were split and expanded in fresh matrigel tissue flasks.

The vcn/c of transduced cells was calculated using 0.6pg of genomic DNA (= 100,000 hESCs) and plasmid DNA (from 10² to 10⁷ copies) for the standard curve. The Q-PCR was performed as previously described (Aker, M. et al. Extended core sequences from the cHS4 insulator are necessary for protecting retroviral vectors from silencing position effects. Hum Gene Ther (2007)) using the eGFP primers shown in Supplementary Table 1.

### Cell Lines and Culture Media

293T cells (CRL11268; American Type Culture Collection; Rockville, MD) were grown in Dulbecco's Modified Eagle Medium (DMEM, Invitrogen, Edinburgh, Scotland) with GlutaMAX^{™} and supplemented with 10% Fetal Bovine Serum (FBS) (PAA Laboratories GmbH, Austria). hMSCs were obtained from Biobanco del Sistema Sanitario Publico de Andalucia (Granada, Spain) and cultured as previously described (Carrillo-Galvez, A.B. et al. Mesenchymal stromal cells express GARP/LRRC32 on their surface: effects on their biology and immunomodulatory capacity. Stem Cells (2015)). Briefly hMSCs were cultured in advanced Dulbecco's modified Eagle's medium (DMEM) supplemented with 10% fetal calf serum (FCS) (Invitrogen, Carlsbad, CA), Glutamax (GIBCO, Grand Island, NY, www.lifetechnologies.com), and 100 U/ml penicillin/streptomycin (GIBCO). All experiments using human samples were performed according to the Institutional Guidelines and approved by the ethics committee of the H.U. Virgen de Macarena.AND-1 (Spanish Stem Cell Bank.)⁷⁵ and H9 (Wicell Research Institute Inc. Madison, WI) hESC lines were maintained undifferentiated in a feeder-free culture as previously described (Munoz, P. et al. Specific marking of hESCs-derived hematopoietic lineage by WAS-promoter driven lentiviral vectors. PLoS ONE (2012)). Briefly hESCs were culture in matrigelcoated T25 flasks and fed daily with hMSC-conditioned medium (Ramos-Mejia, V. et al. Maintenance of Human Embryonic Stem Cells in Mesenchymal Stem Cell-Conditioned Media Augments Hematopoietic Specification. Stem Cells Dev (2012)) supplemented with 8 ng/ml bFGF (Miltenyi Biotech, Bergish Gladbach, Germany). Approval from the Spanish National Embryo Ethical Committee was obtained to work with hESCs.

### Adipocyte and Osteocyte differentiation.

Untransduced hMSCs as well as CEETnI2- and CEETnl2ls2 transduced hMSC were differentiated to adipocytes and osteocytes as previously described (Anderson, P. et al. CD105 (endoglin)-negative murine mesenchymal stromal cells define a new multipotent subpopulation with distinct differentiation and immunomodulatory capacities. PLoS ONE (2013)). Briefly, hMSCs were plated in 6-well plates at a density of 20,000 cells/cm² for adipogenesis, 10,000 cells/cm² for osteogenesis and incubated in the adipogenic and osteogenic MSCs differentiation BulletKits respectively (lonza, Basel, Switzerland).

### Human ESCs hematopoietic differentiation in OP9 co-culture system.

Hematopoietic differentiation was induced as described by Ji et al (Ji, J. et al. OP9 stroma augments survival of hematopoietic precursors and progenitors during hematopoietic differentiation from human embryonic stem cells. Stem Cells (2008)). Briefly, the hESCs lines were transferred onto OP9 feeders for 15 days. To evaluate hematopoietic differentiation and eGFP expression at the different days, cells were dissociated with collagenase IV and Tryple (Gibco), resuspended in FACS buffer, filtered through a 70µm cell strainer (BD Biosciences, Bedford, MA) and stained with anti-mouse CD29-FITC (AbD Serotec, Raleigh, GBK), anti-human CD34-PE-Cy7 and anti-human CD45-APC(all from eBiosciences, San Diego, CA) and analyzed in a FACS Canto II flow cytometer.

### Differentiation of hESCs towards the mesenchymal lineage.

Derivation of MSC-like cell was carried out as previously described (Liu et al., 2012), Briefly, AND-I hESC were treated with 10 µM ROCK for 1 hours, and disaggregated into a single cells with trypsin. The cells were collected in maintenance medium (Embryonic fibroblast conditioned medium supplemented with 4 ng/ml FGF) (Invitrogen), and seeded at a density of 15000/cm2 in Type I collagen coated well tissue culture. 24 hours later, the maintenance medium was supplemented with an equal volume of basal α-MEM (invitrogen) with 10 % FBS (Hyclone), 100 U/ml penicillin, 100 µg/ml streptomycin (Invitrogen) and 50 µM magnesium L-ascorbic acid phosphate (Sigma-Aldrich). 10 days later, the medium was replaced with α-MEM (Invitrogen), supplemented with 100 U/ml penicillin and 100 µg/ml streptomycin, 2 mM L-glutamine. The third passage was used for flow cytometry analysis.

### FACS analysis and FACS-sorting.

- *eGFP expression of total populations:* The different cell types cultured in the presence of absence of Dox (0.1 µg/ml) were resuspended in PBS+FBS+EDTA buffer (FACS buffer) and filtered through a 70 µm cell strainer (BD Biosciences, Bedford, MA). Live cells identifier by 7-AAD viability dye exclusion were analyzed for eGFP expression using a FACS Canto II flow cytometer equipped with the FACS Diva analysis software (BD Bioscience).
- *eGFP expression in hESCs-derived subpopulations.* hMSCs derived from untransduced and transduced hESCs were resuspended in FACS buffer and stained with anti-CD105-APC antibodies (eBioscience, San Diego, CA) and analyzed by FACS. eGFP expression was measured in the CD105⁺ population. Similarly, hematopoietic cells derived from untransduced and transduced hESCs were resuspended in FACS buffer and stained with anti-CD34-PE-Cy7 (BD Biosciences) and anti-CD45-APC (Miltenyi Biotech, Bergish Gladbach, Germany) antibodies. Live cells identified by 7-AAD viability dye exclusion were analyzed for surfacemarker and eGFP expression using a FACS Canto II. The populations analyzed were hemogenic progenitors (CD45⁻CD34⁺), hematopoietic progenitors (CD45⁺CD34⁺), and mature blood cells (CD45⁺CD34⁺).
- *Analysis of pluripotency markers in untransduced and transduced hESCs.* Untransduced and transduced hESCs were stained with the antibodies OCT3/4 (BD Pharmingen), SSE3, TRA-1-60, TRA-1-81 (eBiosciences) and live cells identified by 7-AAD viability dye exclusion (eBiosciences)(data not shown). Samples were acquired and analyzed in a FACS Canto II flow cytometer equipped with the FACS Diva analysis software (Becton Dickinson, Franklin Lakes, NJ).

Separation of eGFP⁺hMSCs and hESCs.Dox-induced eGFP⁺ populations of CEETnl2 and CEETnI2Is2-transduced hMSCs or hESCs were washed and separated using fluorescenceactivated cell sorting (FACS) Aria II Flow Cytometer. A MOI = 0.4 was used to keep the percentage of transduced hMSCs below 15%. For hESCs a MOI = 30 was used to achieve over 30% eGFP⁺ cells and good expression levels for sorting.

### Western Blot

Cells were lysed in RIPA buffer (Sigma) containing protease inhibitors (Sigma Aldrich) at 1 × 10⁶ cells/100 µl. The lysates (20 µg/sample) were resolved by sodium dodecyl sulphatepolyacrylamide gel electrophoresis (10% polyacrylamide under reducing conditions) and electrotransferred to nitrocellulose membranes (Bio-Rad Hercules CA.). Milk-blocked Membranes were probed for 1 hour at room temperature with mouse anti-Tet-repressor (Mobitec; no TET02) at 1:500 dilution at 4°C, and rabbit anti-alpha-tubulin (Santa Cruz, sc-5546). Combination of IRDye 680LT Goat anti-Rabbit IgG (Licors: no 26-68021) and IRDye 800CW Goat anti-Mouse IgG (Licors: no 926-32210) were use at 1:10.000 dilutions to analyzed TetR and tubulin using an Odyssey Image scanner system (LI-COR Biosciences).

### Determination of fold induction and leaking index.

Transduced cells incubated in the presence or absence of Dox (0.1µg/ml) were analyzed by flow cytometry to determine the percentage and Mean Fluorescence Intensity (MFI) of eGFP positive cells as well as the MFI of WLC (WLC = P1 defined as the 7-AAD negative population). The fold induction was calculated using the following formula MFI P1 (+Dox) / MFI P1 (-Dox). Similarly, the *Leakiness* of the system was determined by using the following formulas [% eGFP⁺ (-Dox) x 100 / %eGFP⁺(+Dox)] or [[% eGFP⁺ (-Dox) x 100 / %eGFP⁺(+Dox)]] x MFI eGFP⁺(-Dox)). Background(%eGFP⁺ cells in MOCK transduced cells) were subtracted to the % eGFP⁺ obtained in the different transductions and conditions. Therefore, when the %eGFP⁺ (-Dox) is identical in Mock and Transduced cells the leaking is zero and when the %eGFP⁺ (-Dox) is identical to the %eGFP⁺(+Dox) the leaking is 100.

### DNA methylation assay

The promoter methylation was assessed by bisulfite treatment of Genomic DNA and sequencing of the resulting converted gDNA. In the presence of sodium bisulfite, only the unmethylated cytosines are chemically converted to uracil. The genomic DNA was isolated from hESCs at the desired time points using the DNaseasy kit (Qiagen, Crawly, UK). Sodium bisulfite treatment of genomic DNA was performed using the EpiTect bisulfit kit (Qiagene), according to the manufacturer's instruction. The converted gDNA was used for a nested PCR amplification of SFFV and EF1a promoters. The primers used for each amplification is shown in Table 1. The PCR product band was purified and cloned into PCR 2.1 (Invitrogen) according to the manufacturer's instruction. 15-30 clones were randomly selected and sequenced with M13 primers. Identical sequences were eliminated from the study to avoid duplicated clones.

**Table 1 Primers used for the methylation profile analysis and determination of vector copy number per cell (vcn/c).**

| | |
|---|---|
| SFFVf1 | 5'-TAGAAAAAGGGGGGAATGAAA-3' |
| eGFPr1 | 5'-AAACAACTCCTCACCCTTACTCAC-3' |
| SFFVf2 | 5'-GGGGGAATGAAAGATTTTATTTG-3' |
| eGFPr2 | 5'-CCCTTACTCACCATAATTTCAACC-3' |
| EF1αf1 | 5'-GATGGATAAAGTTTTAAAT-3' |
| EF1αr1 | 5'-CTATTCTTTCCCCTACACTATAC-3' |
| EF1αf2 | 5'-AAGTTTTAAATAGAGAGGAA-3' |
| EF1αr2 | 5'-CTACACTATACCCCCCAATCC-3' |
| TetRf | 5'-GGGATCCTAGTGATTATGTCT-3' |
| TetRr | 5'-TTACGGGTTGTTAAACCTTC-3' |
| GADPHf | 5'-GAAGGTGAAGGTCGGAGTC-3' |
| GADPHr | 5'-GAAGATGGTGATGGGATTTC-3' |
| eGFPfw | 5'-GTTCATCTGCACCACCGGCAAG-3' |
| eGFPrev | 5'-TTCGGGCATGGCGGACTTGA-3' |

### Analysis of the Adipocyte and Osteocyte differentiation:

The different cells line were incubated in osteogenic and adipogenic MSCs differentiation BulletKit media respectively (lonza, Basel, Switzerland), and stained with Oil red (Amresco, solon, OH) to stain lipid vesicles present in adipocytes and with Alizarin Red S (Sigma) to stain calcium deposits in osteocytes.

### mRNA analysis by RT-qPCR.

Total RNA from hMSCs transduced with CEST!n2!s2 or with CEETLN2ls2 was isolated using the Trizol reagent (Invitrogen) and reverse-transcribed using the Superscript first-strand system (Invitrogen). qPCRs were performed using the QuantiTect SYBRGreen PCR kit (Qiagen) on a Stratagene MX3005P system (Agilent Technologies, Santa Clara, CA). Q-PCR reactions consisted of 40 cycles at 94 °C (15 sec), then 60 °C (30 sec) and 72 °C (30 Sec). TetR and GADPH specific primers are shown in Table 1. The relative expression was calculated using ΔΔCT method.

### Statistical Analysis

All data are represented as mean +/- (SEM). The statistical analysis was performed using the GraphPad Prism software (GraphPad Software, Inc., La Jolla, CA) applying the unpaired twotailed t-test. Statistical significance was defined as a P value <0.05.

### Results

### Example 1. Development of an all-in-one LVs system with lower leaking and enhanced inducibility: The Lent-On-Plus system.

In order to generate Tet-On all-in-one LVs that tightly regulate transgene expression in human stem cells we have constructed several LVs harboring different modifications (Fig. 2a). All the constructs express eGFP through the CMV-TetO promoter but have different backbones configurations to increase TetR levels in the nuclei, to avoid promoter silencing and to shield the LVs from enhancers present nearby the integration sites. The original TetR proteins translocate poorly to the nucleus (Ogueta S.B. et al., Design and in vitro characterization of a single regulatory module for efficient control of gene expression in both plasmid DNA and a selfinactivating lentiviral vector. Mol Med (2001)), a crucial step to achieve transcriptional repression. We initially tested two different nuclear localization signals and selected the TetRnl2 as the best repressor to be used in our LV system (Fig.1).

Next, we constructed four all-in-one LVs harboring different promoters and insulator combinations (Fig. 2a); The CESTnl2 and CEETnl2 LVs express the TetRnl2 through the Spleen Focus Forming Virus (SFFV) and the human eukaryotic translocation elongation factor 1α (hEF1α) promoters respectively. The CESTnl2ls2 and CEETnl2ls2 LVs have the same backbone as the CESTnl2 and CEETnl2 respectively but including the insulator Is2 (Benabdellah, K. et al. A chimeric HS4-SAR insulator (IS2) that prevents silencing and enhances expression of lentiviral vectors in pluripotent stem cells. PLoS ONE (2014)). The LVs expressing the TetR through the hEF1α promoter (CEETnl2 and CEETnl2ls2) were named Lent-On-Plus. The fold induction [MFI (+Dox) / MFI (-Dox)] and leaking ([%eGFP⁺(-Dox) *100/%eGFP⁺(+Dox)] of the different LVs were tested first in 293T using a multiciplicity of infection (MOI) of 0.3. At this MOI most of the transduced cells harbored only one vector integrated in their genome, a circumstance where the original CEST had high leaking and poor fold induction (Benabdellah, K., et al. Development of an all-in-one lentiviral vector system based on the original TetR for the easy generation of Tet-ON cell lines. PLoS ONE (2011) and Figs. 2b and 2c).

Expression of the TetRnl2 through the hEF1α promoter in 293T cells decreased the leaking of the vectors in the absence of Dox (Compare CESTnl2 versus CEETnl2 in Fig. 2b, top plots and Fig. 2c, right graph) but also reduced the fold induction (Compare CESTnl2 versus CEETnl2 in Fig. 2c, left graph). It is worth mentioning that, in the presence of Dox, the CEETnl2 LV expressed lower eGFP levels compared to the CESTnl2 LV (Fig. 2b; MFI of the whole living cells (WLC) of CESTnl2 = 4652 versus CEETnl2 = 1976).

In spite of the improvements in leaking and fold induction of the CEETnl2 and CESTnl2 LVs, both vectors still presented high leaking and poor fold induction. Insulators, such as the chicken hypersensitive site 4 (cHS4) (Chung, J.H., et al. A 5' element of the chicken beta-globin domain serves as an insulator in human erythroid cells and protects against position effect in Drosophila. *Cell* (1993)) can shield integrative vectors from nearby regulatory domains favoring a more autonomous regulation and also act as barriers to avoid silencing (Emery, D.W. et al. A chromatin insulator protects retrovirus vectors from chromosomal position effects. Proc Natl Acad Sci U S A (2000); Rivella, S. et al. The cHS4 insulator increases the probability of retroviral expression at random chromosomal integration sites. J Virol (2000)).

SARs/MARs elements have also been incorporated into retroviral vectors improving their transgene expression and preventing promoter inactivation (Agarwal, M. et al. Scaffold attachment region-mediated enhancement of retroviral vector expression in primary T cells. J Virol (1998); Dang, Q. et al. Human beta interferon scaffold attachment region inhibits de novo methylation and confers long-term, copy number-dependent expression to a retroviral vector. J Virol (2000); Ramezani, A. et al. Performance- and safety-enhanced lentiviral vectors containing the human interferon-beta scaffold attachment region and the chicken beta-globin insulator. Blood (2003)). We have previously constructed the Is2 insulator (combining the HS4-650 and a synthetic SAR element) that was able to enhance expression and avoid silencing of LVs in hESCs (Benabdellah, K., A chimeric HS4-SAR insulator (IS2) that prevents silencing and enhances expression of lentiviral vectors in pluripotent stem cells. PLoS ONE (2014)). We hypothesized that the inclusion of this element in the CESTnl2 and CEETnl2 LVs could improve their performance.

As expected, the CESTnl2ls2 LV showed improved expression levels compared to their Is2-negative counterpart (CESTnI2) in the presence of Dox (Fig. 2b; MFI of WLCs CESTnl2ls2 = 31695 versus 4652 in CESTnI2). The higher expression levels of the Is2-containing LVs rendered also a higher fold induction of these LVs (Fig. 2c, left graph). However, this enhancement of expression was not observed when the Is2 was incorporated in the CEETnl2 backbone (MFI= 1976 versus 1214 of CEETnl2 versus CEETnl2ls2 respectively). Yet, the fold induction of the CEETnl2ls2 LVs increased compared to the CEETnl2 LVs (Fig. 1c, left graph) due to the lower leaking in the absence of Dox (Fig. 2c, right graph). In terms of leaking, the effect of the ls2 element has the same tendency in both backbones but is significant only on the CEETnl2 LVs.

Leaking of a regulatable system can be analyzed in different ways. For simplicity, we have used the formula; leaking = [%eGFP⁺(-Dox) *100/%eGFP⁺(+Dox) that generate values from 0 (no leaking) to 100 (absence of regulation). However, this analysis does not discriminate between partially-responsive cells from those cells that do not respond to Dox. We have therefore reanalyzed the leaking using an alternative formula; leaking = [% eGFP⁺ (-Dox) / %eGFP⁺(+Dox)] x MFI eGFP⁺cells (-Dox)] to take into account the expression levels of the transduced cells in the absence of Dox (Fig.3). This analysis confirmed that the nI2 signal reduced the leaking of the CEST LVs and that the Is2 element reduced leaking only in the CEET LV backbone.

All together these data indicate that only the CEETnl2ls2 LV was able to regulate transgene expression in 293T harboring one vector copy number per cell (vcn/c). As a down side, we detected a reduction on the titer (2-3 fold) of the Is2-LVs compared to their Is2-negative counterparts (data not shown).

The fold induction parameter indicates the increment of eGFP expression of the total population upon the addition of Dox (see M&M for details). Since we kept transduction below 40% (to obtain cell populations harboring only one vcn/c), the fold induction is underestimated. To study the potency of the system we further analyzed the inducibility of the Lent-On-Plus LVs (CEETnl2 and CEETnl2ls2) on cell populations that are 100% responsive to Dox (Fig. 4). These data confirmed the positive effect of the Is2 insulator on leaking (Fig. 4 CEETnl2 = 66.2 versus CEETnl2ls2 = 7.4) and fold induction (CEETnI2 = 5.7 versus CEETnl2ls2 = 38.8) in the Lent-On-Plus LVs (CEET backbone).

### Example 2. The Is2+ Lent-On-Plus LVs efficiently regulate transgene expression in human mesenchymal stromal cells.

We next tested the new inducible systems in hMSCs. We transduce hMSCs with the CESTnl2, CESTnl2ls2, CEETnl2 and CEETnl2ls2 LVs at a MOI=1 and 10 days later analyzed for GFP expression in the presence or absence of Dox (Fig. 5a). As observed in 293T cells, the expression of the TetRnl2 through the hEF1α promoter (Lent-On-Plus LVs) reduced the leaking of the LVs (Fig. 5a right top plots) even at low vcn/c. Importantly, the combination of the hEF1α promoter and the Is2 insulator blocked the expression of eGFP almost completely in the absence of Dox (Fig. 5a, compare untransduced (top-left plot) with CEETnI2Is2-transduced (top-right plot) hMSCs). On the contrary, the vectors expressing TetRnl2 through the SFFV promoter (CESTnI2 and CESTnI2Is2) had high leaking at low vcn/c (Fig. 5a, top-left plots). Interestingly, the CEETnl2 LVs express even lower TetR levels than CESTnl2 LVs in MSCs (Fig. 6) which indicates that the lower leaking of the CEETnl2 is not due to higher expression levels of the repressor but to the Lent-On-Plus backbone configuration. Based on these results we focused on the CEETnl2 and CEETnl2ls2 (Lent-On-Plus systems) for later studies.

Inducible systems should be able to maintain transgene regulation upon cellular expansion and/or differentiation. We therefore transduced hMSCs with the CEETnl2 and CEETnl2ls2 LVs and studied transgene regulation after expansion (Fig. 5b, Top panels) and differentiation toward the adipogenic(Fig. 5b, middle panels) and osteogenic (Fig. 5b,bottom panels) lineages (Fig. 7 shows the characterization of differentiated cells). In all cases, the CEETnl2ls2 had the lowest leaking and higher fold induction as can be observed in the representative plots (Fig. 5b, left) and in the graphs (Fig. 5b, right). It is remarkable that neither the long-term culture nor the differentiation of the hMSCs toward the adipogenic or osteogenic lineages influenced the low leaking and inducibility of the CEETnl2ls2 LVs in this cell type. As in 293T cells, leaking of the different LVs were also re-analyzed to take into account the MFI. The new analysis showed similar conclusions but improved significance of some results (Fig. 8).

Finally we aimed to generate pure populations of CEETnl2 and CEETnI2Is2- cells harboring 1 vcn/c. hMSCs were transduced at low MOI to achieve 9-15% eGFP⁺ cells upon induction with Dox. Two weeks later eGFP⁺hMSCs were sorted to a purity of 99% (Fig. 9a), cultured for 8 days in the absence of Dox and then analyzed for responsiveness to Dox (Fig. 9b). The results confirmed the positive effect of the Is2 insulator on leaking (CEETnl2 = 54 versus CEETnl2ls2 = 2.6) and fold induction (CEETnl2 = 2.8 versus CEETnl2ls2 = 14) in hMSCs. Since in these populations all cells contain only one vector integrated in their genome, the good fold induction obtained with the CEETnl2ls2 (14) represent the average Dox response of one Lent-On-Plus LV integrated in hMSCs. Importantly, the leaking of the CEETnl2ls2 LVs remains very low (2.8) with only a minor increment in MFI of the WLC compared to the untransduced population (Fig. 9b: MFI of WLC Mock = 85 versus MFI of WLCs CEETnl2ls2 -Dox = 117)

### Example 3.Generation of doxycycline-responsive pluripotent stem cells by the CEETnl2 and the CEETnI2Is2.

The generation of pluripotent stem cells expressing the desired transgene under the tight control of an inducer has been a very difficult task to achieve due to the low efficiency of existing delivery methods, the strong silencing of the transgenes and the side effects of the transactivators. We hypothesized that the use of insulated LVs expressing the TetRnl2 through stable promoters could overcome previous existing limitations to generate Dox-inducible hESCs. To investigate this possibility, we first tested the Dox-responsiveness of two hESCs lines (AND-1 and H9) transduced with the CESTnl2, CESTnl2ls2, CEETnl2 and CEETnl2ls2 LVs at MOI=5 (Fig.10). Neither, the CESTnl2 nor CESTnl2ls2 LVs could regulate transgene expression in hESCs (Fig. 10a, left plots). On the contrary, both Lent-On-Plus LVs expressing TetRnl2 through the hEF1α promoter (CEETnl2 and CEETnl2ls2) achieved low leaking (Fig. 10a; compare Mock versus CEETnI2- and CEETnI2Is2- transduced hESCs) and good transgene expression in the presence of Dox at low vcn/c (Fig. 10a, right plots and Fig. 11). However, it is important to highlight that not all transduced-hESCs will express eGFP in the presence of Dox as can be observed in Fig. 10a. Indeed, only around 20% of the hESCs express eGFP in spite of having an average of 1 vcn/c. This observation has been described previously and is probably due to transcriptional variegation of the integrated LVs. We next studied whether CEETnI2- and CEETnI2Is2-transduced hESCs maintained transgene regulation upon cellular expansion. We therefore cultured the inducible hESCs cells in the presence or absence of Dox for 33 days and analyzed their leaking and fold induction at different time points (Fig. 10b). Both vectors kept their Dox-responsiveness (fold induction) during time in culture without significant changes (Fig. 10b, left graph) and, as observed in hMSCs, the presence of the Is2 insulator reduced the leaking of the CEET LVs (Fig. 10b, right graph). However, contrary to what was observed in MSCs, the CEETnl2ls2 LVs have similar fold induction compared to the CEETnl2 LVs (Fig. 10b, left graph). We additionally showed that we can turn off and turn on transgene expression in the CEETnI2- and CEETnI2Is2-transduced hESCs by withdrawal or addition of Dox (Fig. 10c).

We next generated sorted CEETnI2- and CEETnI2Is2-transduced hESCs in order to study Dox responsiveness in a purer population. hESCs were transduced at MOI=30 and two weeks later, CEETnI2- and CEETnI2Is2-transduced hESCs were sorted to a purity of 74 and 50% respectively (Fig. 12a). Sorted cells were cultured for 8 days in the absence of Dox and Tra-1-81⁺/SSE3⁺hESCs analyzed for responsiveness to Dox (Fig. 12b). The results corroborate the positive effect of the Is2 insulator on leaking (CEETnl2 = 4.7 versus CEETnl2ls2 = 1.3) but not in fold induction (CEETnl2 = 5.5 versus CEETnl2ls2 = 5.2).

### Example 4. The hEF1α promoter is a key factor for regulation of the CEETnl2 and CEETnI2Is2 LVs.

Transgene silencing due to promoter methylation of integrative RVs has been a major problem for stable expression in hESCs. Several studies have shown that pluripotent cells can block expression of exogenous retroviral elements by complex defense mechanisms (Cherry, S.R. et al. Retroviral expression in embryonic stem cells and hematopoietic stem cells. Mol Cell Biol (2000)). It has been previously described that the SFFV promoter is silenced through methylation in human pluripotent cells (Herbst, F. et al. Extensive methylation of promoter sequences silences lentiviral transgene expression during stem cell differentiation in vivo. Mol Ther (2012)) while the hEF1α promoter is highly resistant allowing stable transgene expression in these cell types (Hong, S. et al. Functional analysis of various promoters in lentiviral vectors at different stages of in vitro differentiation of mouse embryonic stem cells. Mol Ther (2007); Wang, R. et al. Promoter-dependent EGFP expression during embryonic stem cell propagation and differentiation. Stem Cells Dev (2008); Xia, X. et al. Transgenes delivered by lentiviral vector are suppressed in human embryonic stem cells in a promoter-dependent manner. Stem Cells Dev (2007)). We therefore analyzed the methylation profiles of the SFFV and hEF1α promoters in CESTnI2- and in CEETnl2-transduced hESCs (Fig. 13a and 13b respectively). As expected by the low leaking and good inducibility of the CEETnl2 (Fig. 12b and Fig. 13b, plots), the hEF1α promoter presented a very low degree of methylation (Fig. 13b, bottom panel). On the contrary, the SFFV promoter in the CESTnl2 LVs was highly methylated (>90%; Fig. 13a, bottom panel) which resulted in the lost of transgene regulation (Fig. 13a, top plots). Of note, the presence of Is2 insulator in the CESTnl2ls2 LVs was not able to avoid SFFV methylation (data not shown). We further corroborated the influence of promoter methylation on TetR expression levels by analyzing CESTnl2- and CEETnl2-transduced hESCs by western blot (Fig. 13c). Only the CEETnl2-transduced hESCs expressed detectable TetR protein as was expected by the absence of regulation in these cells and by the methylation of the SFFV promoter in the CESTnl2 LVs.

### Example 5. Doxycycline responsiveness of the Lent-On-Plus LVs is maintained after differentiation of hESCs toward the mesenchymal and hematopoietic lineages.

Ideally, the inducible systems for pluripotent stem cells must be also able to maintain drug responses upon differentiation toward different cell types. We then analyzed whether the Lent-On-Plus systems (CEETnl2 and CEETnl2ls2) maintained the Dox responsiveness in mesenchymal and hematopoietic cells derived from transduced hESCs (Fig. 14).
hESCs transduced with the CEETnl2 and CEETnl2ls2 LVs at high MOI (vcn/c of 2.5 and 1.9 respectively) (Fig. 14a) were differentiated toward hMSCs-like cells as described previously (Sanchez, L. et al. Enrichment of human ESC-derived multipotent mesenchymal stem cells with immunosuppressive and anti-inflammatory properties capable to protect against experimental inflammatory bowel disease. Stem Cells (2011)) (see M&M for details). At day 10 of differentiation, homogeneous fibroblast-like cells were obtained and at this point, Dox was added and eGFP expression levels analyzed in the CD105⁺ (a hMSCs marker) population (Fig. 14b). Our data showed that hMSCs-derived from CEETnl2-transduced hESCs had low leaking (compare Mock- with CEETnI2- and CEETnI2Is2-transduced cells in Fig. 14b) and a good expression levels after Dox addition (Fig. 14b, right plots). These results indicate that the expression of the TetRnl2 repressor through the hEF1α promoter was enough to achieve stable transgene regulation even after hESCs differentiation into hMSCs. The high titer of the CEETnl2 allowed high transduction efficiency of hESCs (Fig. 14a) achieving up to 98% eGFP⁺ cells upon hMSC differentiation (Fig. 14b). The CEETnl2ls2 LVs were less efficient (56%) due to the lower titer, but the CEETnI2Is2-hESCs derived hMSCs were more tightly regulated (lower leaking) than CEETnI2-hESCs derived hMSCs (Fig. 14b, upper versus lower plots; -Dox). Therefore, the presence of the Is2 insulator is required for a tight control of gene expression in hESCs-derived hMSCs which is in agreement to what was observed in hMSCs (Fig. 5 and Fig. 9).

We finally differentiated CEETnI2- and CEETnI2Is2- transduced hESCs toward the hematopoietic lineage using the OP9 differentiation protocol as previously described³³ (see M&M for details). The cells were maintained in the presence or absence of Dox from day 1 of differentiation and the eGFP expression was analyzed in the different hematopoietic populations at day 8 and day 15 (Fig. 14c). The CD45⁻CD34⁺ population (hemato-endothelial progenitors) were analyzed at day 8 (Fig. 7c, left panels), the more mature CD45⁺CD34⁻ hematopoietic cells (Benabdellah, Ket al., A chimeric HS4-SAR insulator (IS2) that prevents silencing and enhances expression of lentiviral vectors in pluripotent stem cells. PLoS ONE (2014); Ji, J. et al. OP9 stroma augments survival of hematopoietic precursors and progenitors during hematopoietic differentiation from human embryonic stem cells. Stem Cells (2008)) at day 15 (Fig. 14c, right panels) and the CD45⁺CD34⁺ cells (hematopoietic progenitors) were analyzed both days. Both Lent-On-Plus LVs showed very low leaking in the absence of Dox (compare Mock- with CEETnI2- and CEETnI2Is2- transduced cells in Fig. 14c) and good expression in its presence in all hematopoietic populations analyzed (CD45⁻CD34⁺, CD45⁺CD34⁺ and CD45⁺CD34⁻). The presence of the Is2 insulator in the CEETnl2ls2 had only a minor effect in the leaking of these populations compared to the CEETnl2-transduced cells (Fig. 14c, -Dox plots). Interestingly, the CEETnl2 outperformed the CEETnl2ls2 in terms of percentage of transduced cells, in part due to its better titer (Fig. 14c, +Dox plots, compare CEETnl2 versus CEETnI2Is2).

### SEQUENCE LISTING

<160> NUMBER OF SEQ ID NOS: 3
<210> SEQ ID NO 1
<211> LENGTH: 367
<212> TYPE: DNA
<213> ORGANISM: Artificial Sequence
<220> FEATURE:
   <223> OTHER INFORMATION: Synthetic S/MAR nucleic acid molecule
   containing 5 M /SARs recognition signatures
<220> FEATURE:
<221> NAME/KEY: misc_feature
<222> LOCATION: (I)..(367)
<400> SEQUENCE: 1 
(MRS)
taaataaacttataaattgtgagagaaattaatgaatgtctaagttaatgcagaaacgga 60
ggctcctcatttatttttgaacttaaagacttaatattgtgaaggtatactttctttaat 120
aataagcctgcgcccaatatgttcaccccaaaaaagctgtttgttaacttgtcaacctca 180
tttaaaatatataagaaacagcccaaagacaataacaaaagaataataaaaagaatgaa 240
atatgtaattctttcagagtaaaaatcacacccatgacctggccactgagggcttgatca 300
attcactttgaatttggcattaaataccattaaggtatattaactgattttaaaataaga 360
tatattc 367
<210> SEQ ID NO 2
<211> LENGTH: 655
<212> TYPE: DNA
<213> ORGANISM: Artificial Sequence
<220> FEATURE:
   <223> OTHER INFORMATION: Nucleic acid molecule HS4-650 bp
<220> FEATURE:
<221> NAME/KEY: misc_feature
<222> LOCATION: (I)..(655
<400> SEQUENCE: 2 
aagatctgctcacggggacagcccccccccaaagcccccagggatgtaattacgtccctc 60
ccccgctagggggcagcagcgagccgcccggggctccgctccggtccggcgctccccccg 120
catccccgagccggcagcgtgcggggacagcccgggcacggggaaggtggcacgggatcg 180
ctttcctctgaacgcttctcgctgctctttgagcctgcagacacctgggggggatacgggg 240
aaaatgtgtctgagcctgcatgtttgatggtgtctggatgcaagcagaaggggtggaaga 300
gcttgcctggagagatacagctgggtcagtaggactgggacaggcagctggagaattgcc 360
atgtagatgttcatacaatcgtcaaatcatgaaggctggaaaagccctccaagatcccca 420
agaccaaccccaacccacccaccgtgcccactggccatgtccctcagtgccacatcccca 480
cagttcttcatcacctccagggacggtgaccccccacctccgtgggcagctgtgccact 540
gcagcaccgctctttggagaaggtaaatcttgctaaatccagcccgaccctcccctggca 600
caacgtaaggccattatctctcatccaactccaggacggagtcagtgagaatatt 655
<210> SEQ ID NO 3
<211> LENGTH: 1022
<212> TYPE: DNA
<213> ORGANISM: Artificial Sequence
<220> FEATURE:
   <223> OTHER INFORMATION: IS2
<220> FEATURE:
<221> NAME/KEY: misc_feature
   <222> LOCATION: (I)..(I022)
<400> SEQUENCE: 3 
aagatctgctcacggggacagcccccccccaaagcccccagggatgtaattacgtccctc 60
ccccgctagggggcagcagcgagccgcccggggctccgctccggtccggcgctccccccg 120
catccccgagccggcagcgtgcggggacagcccggcacggggaaggtggcacgggatcg 180
 
ctttcctctgaacgcttctcgctgctctttgagcctgcagacacctgggggatacgggg 240
aaaatgtgtctgagcctgcatgtttgatggtgtctggatgcaagcagaaggggtggaaga 300
gcttgcctggagagatacagctgggtcagtaggactgggacaggcagctggagaattgcc 360
atgtagatgttcatacaatcgtcaaatcatgaaggctggaaaagccctccaagatcccca 420
agaccaaccccaacccacccaccgtgcccactggccatgtccctcagtgccacatcccca 480
cagttcttcatcacctccagggacggtgacccccccacctccgtgggcagctgtgccact 540
gcagcaccgctctttggagaaggtaaatcttgctaaatccagcccgaccctcccctggca 600
caacgtaaggccattatctctcatccaactccaggacggagtcagtgagaatatttaaat 660
aaacttataaattgtgagagaaattaatgaatgtctaagttaatgcagaaacggaggctc 720
ctcatttatttttgaacttaaagacttaatattgtgaaggtatactttctttaataataa 780
gcctgcgcccaatatgttcaccccaaaaaagctgtttgttaacttgtcaacctcatttaa 840
aatatataagaaacagcccaaagacaataacaaaagaataataaaaaagaatgaaatatg 900
taattctttcagagtaaaaatcacacccatgacctggccactgagggcttgatcaattca 960
ctttgaatttggcattaaataccattaaggtatattaactgattttaaaataagatatat 1020
tc 1022
 SEQ ID NO 4

ARKCLQAGMNLEARKTKKK

gcccgaaaatgtcttcaggctggaatgaacctggaagctcgaaaaacaaagaag

## Claims

1. A lentiviral vector having nucleic acid sequences capable of integrating into the genome of a mammalian cell comprising: a) two regulatory control elements, b) at least two coding nucleic acid molecules referred to as the first coding nucleic acid molecule and as the second coding nucleic acid molecule, operatively associated with the regulatory control elements and capable of expression in the cell, c) a nucleic acid molecule encoding a nuclear localization element, and d) an insulator element,
wherein the regulatory control elements, the coding nucleic acid molecules, and the nucleic acid molecule encoding the nuclear localization element are inserted between the lentiviral LTRs with the U3 deleted, and the insulator element is inserted in place of the U3 of the 3'LTR,
wherein the second coding nucleic acid molecule encodes a protein capable of repressing the regulatory control element that controls the expression of the first coding nucleic acid molecule,
wherein the regulatory control element is inducible and can be regulated by the protein encoded by second coding nucleic acid molecule,
wherein the nucleic acid molecule encoding the nuclear localization signal is associated to the second coding nucleic acid molecule so that both nucleic acid molecules encode a single polypeptide;
wherein the nucleic acid molecule encoding the nuclear localization signal is selected from the list consisting of SEQ ID No 4 or a nucleic acid molecule having at least 90% identity, over the total length of the sequence, to SEQ ID NO 4 and encoding an amino-acid sequence that is part of a larger polypeptide and that is capable to induce the import into the cell nucleus of said polypeptide;
wherein the insulator sequence is selected from the list consisting of:
a. SEQ ID No 1 or a complementary sequence thereof; or a nucleic acid molecule having at least 90% identity, over the total length of the sequence, to SEQ ID NO 1 and capable of shielding the gene cassette integrated in the host genome from the effect of regulatory sequences from the host genome, and vice versa,
b. SEQ ID No 1 associated with SEQ ID No 2 so that they form a single nucleic acid sequence or a complementary sequence thereof; or a nucleic acid molecule having at least 90% identity, over the total length of the sequence, to SEQ ID No 1 associated with SEQ ID No 2 so that they form a single nucleic acid sequence capable of shielding the gene cassette integrated in the host genome from the effect of regulatory sequences from the host genome, and vice versa, or
c. SEQ ID No 3 or a complementary sequence thereof; or a nucleic acid molecule having at least 90% identity, over the total length of the sequence, to SEQ ID No 3 and capable of shielding the gene cassette integrated in the host genome from the effect of regulatory sequences from the host genome, and vice versa; and
wherein the regulatory control element that regulates the expression of the second coding nucleic acid molecule is the human elongation factor-1 alpha (hEF1α) promoter.

2. The lentiviral vector according to claim 1, wherein the insulator element is introduced in the anti-sense orientation.

3. The lentiviral vector according to any of claims 1 to 2, wherein the second coding nucleic acid molecule preferably encodes a regulatory protein whose regulatory function is drug-responsive, preferably tetracycline- or a variant thereof- responsive.

4. The lentiviral vector according to claim 3, wherein the second coding nucleic acid molecule preferably encodes a protein based on the original TetR repressor element.

5. The lentiviral vector according to any of claims 1 to 4, wherein the regulatory control element that regulates the expression of the first coding nucleic acid molecule comprises a binding site for the regulatory protein encoded by the second coding nucleic acid molecule.

6. The lentiviral vector according to claim 5, wherein the second coding nucleic acid molecule encodes a protein that in the absence of the drug mentioned in claim 3, binds to the regulatory control element that regulates the expression of the first coding nucleic acid molecule to inhibit said expression.

7. The lentiviral vector according to any of claims 5 to 6, wherein the regulatory control element that regulates the expression of the first coding nucleic acid molecule further comprises the CMV promoter.

8. The lentiviral vector according to claim 1, wherein the regulatory control element that regulates the expression of the first coding nucleic acid molecule contains a binding site for the regulatory protein encoded by the second nucleic acid molecule and preferably further comprises the CMV promoter; wherein the regulatory control element that regulates the expression of the second coding nucleic acid molecule is constitutively active; wherein the second coding nucleic acid molecule encodes a protein whose function is drug-responsive, preferably tetracycline- or a variant thereof- responsive, more preferably is based on the TetR repressor element; and wherein the nucleic acid molecule encoding the nuclear localization factor preferably encodes the nuclear localization signal from the glucocorticoid receptor named nI2.

9. A composition comprising the lentiviral vector of any of claims 1 to 8.

10. An in vitro method for expressing a nucleic acid molecule in a mammalian cell in a regulatable manner, comprising a) administering to the cell an effective amount of the lentiviral vector of any of claims 1 to 8, and b) expressing the nucleic acid molecules of the genetic construct to produce the coding nucleic acid molecule RNAs and its encoding polypeptides.

11. The method of claim 10, wherein the mammalian cell is a stem cell of embryonic or adult tissue origin.

## Patentansprüche

1. Lentiviraler Vektor aufweisend Nukleinsäuresequenzen, welcher in der Lage ist, sich in das Genom einer Säugetierzelle zu integrieren, umfassend: a) zwei regulatorische Kontrollelemente, b) mindestens zwei kodierende Nukleinsäuremoleküle bezeichnet als das erste kodierende Nukleinsäuremolekül und als das zweite kodierende Nukleinsäuremolekül, welche operativ mit den regulatorischen Kontrollelementen assoziiert sind und in der Lage sind, sich in der Zelle zu exprimieren, c) ein Nukleinsäuremolekül, welches ein Kernlokalisierungselement kodiert, und d) ein Isolierungselement,
wobei die regulatorischen Kontrollelemente, die kodierenden Nukleinsäuremoleküle und das Nukleinsäuremolekül, welches das Kernlokalisierungselement kodiert, zwischen den lentiviralen LTRs mit dem U3 deletiert eingefügt werden, und das Isolierungselement anstelle vom U3 des 3'LTRs eingefügt wird,
wobei das zweite kodierende Nukleinsäuremolekül ein Protein kodiert, welches in der Lage ist, das regulatorische Kontrollelement, welches die Expression des ersten kodierenden Nukleinsäuremoleküls kontrolliert, zu hemmen,
wobei das regulatorische Kontrollelement induzierbar ist und durch das Protein, welches vom zweiten kodierenden Nukleinsäuremolekül kodiert wird, reguliert werden kann,
wobei das Nukleinsäuremolekül, welches das Kernlokalisierungssignal kodiert, mit dem zweiten kodierenden Nukleinsäuremolekül assoziiert ist, sodass beide Nukleinsäuremoleküle ein einziges Polypeptid kodieren;
wobei das Nukleinsäuremolekül, welches das Kernlokalisierungssignal kodiert, aus der Liste bestehend aus SEQ ID NO 4 oder ein Nukleinsäuremolekül mit mindestens 90% Identität, über der gesamten Länge der Sequenz, mit SEQ ID NO 4 und welches eine Aminosäuresequenz kodiert, welche Bestandteil eines größeren Polypeptids ist und in der Lage ist, das Einführen in den Zellkern des genannten Polypeptids zu induzieren, ausgewählt wird;
wobei die Isolierungssequenz aus der Liste bestehend aus:
a. SEQ ID NO 1 oder eine komplementäre Sequenz derselben; oder ein Nukleinsäuremolekül mit mindestens 90% Identität, über der gesamten Länge der Sequenz, mit SEQ ID NO 1 und welches in der Lage ist, die Genkassette, welche in das Wirtsgenom integriert ist, vor dem Effekt von regulatorischen Sequenzen aus dem Wirtsgenom, und umgekehrt, zu schützen,
b. SEQ ID NO 1 assoziiert mit SEQ ID NO 2, sodass sie eine einzige Nukleinsäuresequenz oder eine komplementäre Sequenz derselben bilden; oder ein Nukleinsäuremolekül mit mindestens 90% Identität, über der gesamten Länge der Sequenz, mit SEQ ID NO 1 assoziiert mit SEQ ID NO 2, sodass sie eine einzige Nukleinsäuresequenz bilden, welche in der Lage ist, die Genkassette, welche in das Wirtsgenom integriert ist, vor dem Effekt von regulatorischen Sequenzen aus dem Wirtsgenom, und umgekehrt, zu schützen, oder
c. SEQ ID NO 3 oder eine komplementäre Sequenz derselben; oder ein Nukleinsäuremolekül mit mindestens 90% Identität, über der gesamten Länge der Sequenz, mit SEQ ID NO 3 und welches in der Lage ist, die Genkassette, welche in das Wirtsgenom integriert ist, vor dem Effekt von regulatorischen Sequenzen aus dem Wirtsgenom, und umgekehrt, zu schützen, ausgewählt wird; und
wobei das regulatorische Kontrollelement, welches die Expression des zweiten kodierenden Nukleinsäuremoleküls reguliert, der Promoter des menschlichen Elongationsfaktors-1 alpha (hEF1α) ist.

2. Lentiviraler Vektor nach Anspruch 1, wobei das Isolierungselement in der Antisense-Orientierung eingeführt ist.

3. Lentiviraler Vektor nach einem der Ansprüche 1 bis 2, wobei das zweite kodierende Nukleinsäuremolekül vorzugsweise ein regulatorisches Protein kodiert, dessen regulatorische Funktion eine auf Arzneimittel reagierende, vorzugsweise auf Tetracyclin oder eine Variante desselben reagierende Funktion ist.

4. Lentiviraler Vektor nach Anspruch 3, wobei das zweite kodierende Nukleinsäuremolekül vorzugsweise ein Protein basierend auf dem ursprünglichen TetR-Repressorelement kodiert.

5. Lentiviraler Vektor nach einem der Ansprüche 1 bis 4, wobei das regulatorische Kontrollelement, welches die Expression des ersten kodierenden Nukleinsäuremoleküls reguliert, eine Bindungsstelle für das regulatorische Protein, welches vom zweiten kodierenden Nukleinsäuremolekül kodiert wird, umfasst.

6. Lentiviraler Vektor nach Anspruch 5, wobei das zweite kodierende Nukleinsäuremolekül ein Protein kodiert, welches in Abwesenheit vom in Anspruch 3 erwähnten Arzneimittel, an das regulatorische Kontrollelement bindet, welches die Expression des ersten kodierenden Nukleinsäuremoleküls reguliert, um die genannte Expression zu inhibieren.

7. Lentiviraler Vektor nach einem der Ansprüche 5 bis 6, wobei das regulatorische Kontrollelement, welches die Expression des ersten kodierenden Nukleinsäuremoleküls reguliert, zusätzlich den CMV-Promoter umfasst.

8. Lentiviraler Vektor nach Anspruch 1, wobei das regulatorische Kontrollelement, welches die Expression des ersten kodierenden Nukleinsäuremoleküls reguliert, eine Bindungsstelle für das regulatorische Protein, welches vom zweiten Nukleinsäuremolekül kodiert wird, enthält und vorzugsweise zusätzlich den CMV-Promoter umfasst; wobei das regulatorische Kontrollelement, welches die Expression des zweiten kodierenden Nukieinsäuremoleküls reguliert, konstitutiv aktiv ist; wobei das zweite kodierende Nukleinsäuremolekül ein Protein kodiert, dessen Funktion eine auf Arzneimittel reagierende, vorzugsweise auf Tetracyclin oder eine Variante desselben reagierende Funktion ist, weiter vorzugsweise auf dem TetR-Repressorelement basiert; und wobei das Nukleinsäuremolekül, welches den Kernlokalisierungsfaktor kodiert, vorzugsweise das Kernlokalisierungssignal vom Glukokortikoidrezeptor mit dem Namen nl2 kodiert.

9. Zusammensetzung umfassend den lentiviralen Vektor nach einem der Ansprüche 1 bis 8.

10. In-vitro-Verfahren zur Expression eines Nukleinsäuremoleküls in einer Säugetierzelle auf eine regulatorische Weise, umfassend a) das Verabreichen der Zelle eine wirksame Menge des lentiviralen Vektors nach einem der Ansprüche 1 bis 8, und b) Exprimieren der Nukleinsäuremoleküle des genetischen Konstrukts, um die RNA der kodierenden Nukleinsäuremoleküle und deren kodierenden Polypeptide herzustellen.

11. Verfahren nach Anspruch 10, wobei die Säugetierzelle eine Stammzelle embryonalen Ursprungs oder aus adultem Gewebe ist.

## Revendications

1. Vecteur lentiviral ayant des séquences d'acide nucléique capables d'intégration dans le génome d'une cellule de mammifère comprenant : a) deux éléments de contrôle régulateurs, b) au moins deux molécules d'acide nucléique codant dénommées première molécule d'acide nucléique codant et seconde molécule d'acide nucléique codant, associées opérationnellement avec les éléments de contrôle régulateurs et capables d'expression dans la cellule, c) une molécule d'acide nucléique codant un élément de localisation nucléaire, et d) un élément isolant,
dans lequel les éléments de contrôle régulateurs, les molécules d'acide nucléique codant, et la molécule d'acide nucléique codant l'élément de localisation nucléaire sont insérées entre les LTR lentiviraux avec l'U3 délétionné, et l'élément isolant s'insère à la place de l'U3 du 3'LTR,
dans lequel la seconde molécule d'acide nucléique codant code une protéine capable de réprimer l'élément de contrôle régulateur qui contrôle l'expression de la première molécule d'acide nucléique codant,
dans lequel l'élément de contrôle régulateur est inductible et peut être régulé par la protéine codée par la seconde molécule d'acide nucléique codant,
dans lequel la molécule d'acide nucléique codant le signal de localisation nucléaire est associée à la seconde molécule d'acide nucléique codant de sorte que les deux molécules d'acide nucléique codent un seul polypeptide ;
dans lequel la molécule d'acide nucléique codant le signal de localisation nucléaire est sélectionnée dans la liste consistant en SEQ ID NO 4 ou une molécule d'acide nucléique ayant une identité d'au moins 90%, sur toute la longueur de la séquence, avec SEQ ID NO 4 et codant une séquence d'acides aminés qui fait partie d'un polypeptide plus grand et qui est capable d'induire l'importation dans le noyau de cellule dudit polypeptide ;
dans lequel la séquence de l'isolante est sélectionnée de la liste constituée de :
a. SEQ ID NO 1 ou une séquence complémentaire de celle-ci ; ou une molécule d'acide nucléique ayant une identité d'au moins 90%, sur toute la longueur de la séquence, avec SEQ ID NO 1 et capable de protéger la cassette de gènes intégrée dans le génome hôte de l'effet des séquences régulatrices du génome hôte, et vice versa,
b. SEQ ID NO 1 associée avec SEQ ID NO 2 de sorte qu'elles forment une séquence unique d'acide nucléique ou une séquence complémentaire de celle-ci ; ou une molécule d'acide nucléique ayant une identité d'au moins 90%, sur toute la longueur de la séquence, avec SEQ ID NO 1 associée avec SEQ ID NO 2 de sorte qu'elles forment une séquence unique d'acide nucléique capable de protéger la cassette de gènes intégrée dans le génome hôte de l'effet des séquences régulatrices du génome hôte, et vice versa, ou
c. SEQ ID NO 3 ou une séquence complémentaire de celle-ci ; ou une molécule d'acide nucléique ayant une identité d'au moins 90%, sur toute la longueur de la séquence, avec SEQ ID NO 3 et capable de protéger la cassette de gènes intégrée dans le génome hôte de l'effet de séquences régulatrices du génome hôte, et vice versa ; et
dans lequel l'élément de contrôle régulateur qui régule l'expression de la seconde molécule d'acide nucléique codant est le promoteur du facteur d'élongation humain 1 alpha (hEF1α).

2. Vecteur lentiviral selon la revendication 1, dans lequel l'élément isolant est introduit dans l'orientation anti-sens.

3. Vecteur lentiviral selon l'une quelconque des revendications 1 à 2, dans lequel la seconde molécule d'acide nucléique codant code de préférence une protéine régulatrice dont la fonction régulatrice répond à des médicaments, préférablement répond à tétracycline ou à une variante de celle-ci.

4. Vecteur lentiviral selon la revendication 3, dans lequel la seconde molécule d'acide nucléique codant code de préférence une protéine basée sur l'élément répresseur TetR original.

5. Vecteur lentiviral selon l'une quelconque des revendications 1 à 4, dans lequel l'élément de contrôle régulateur qui régule l'expression de la première molécule d'acide nucléique codant comprend un site de liaison pour la protéine régulatrice codée par la seconde molécule d'acide nucléique codant.

6. Vecteur lentiviral selon la revendication 5, dans lequel la seconde molécule d'acide nucléique codant code une protéine qui, en l'absence du médicament mentionné dans la revendication 3, se lie à l'élément de contrôle régulateur qui régule l'expression de la première molécule d'acide nucléique codant pour inhiber ladite expression.

7. Vecteur lentiviral selon l'une quelconque des revendications 5 à 6, dans lequel l'élément de contrôle régulateur qui régule l'expression de la première molécule d'acide nucléique codant comprend en outre le promoteur de CMV.

8. Vecteur lentiviral selon la revendication 1, dans lequel l'élément de contrôle régulateur qui régule l'expression de la première molécule d'acide nucléique codant contient un site de liaison pour la protéine régulatrice codée par la seconde molécule d'acide nucléique et comprend préférablement en outre le promoteur de CMV ; dans lequel l'élément de contrôle régulateur qui régule l'expression de la seconde molécule d'acide nucléique codant est constitutivement actif ; dans lequel la seconde molécule d'acide nucléique codant code une protéine dont la fonction répond à des médicaments, préférablement répond à tétracycline ou à une variante de celle-ci, plus préférablement est basée sur l'élément répresseur TetR ; et dans lequel la molécule d'acide nucléique codant le facteur de localisation nucléaire code préférablement le signal de localisation nucléaire depuis le récepteur glucocorticoïde dénommé nl2.

9. Composition comprenant le vecteur lentiviral selon l'une quelconque des revendications 1 à 8.

10. Procédé *in vitro* pour exprimer une molécule d'acide nucléique dans une cellule de mammifère d'une manière régulable, comprenant a) l'administration à la cellule d'une quantité effective du vecteur lentiviral selon l'une quelconque des revendications 1 à 8, et b) l'expression des molécules d'acide nucléique de la construction génétique pour produire les ARN de la molécule d'acide nucléique codante et leurs polypeptides codants.

11. Procédé selon la revendication 10, dans lequel la cellule de mammifère est une cellule souche d'origine de tissu embryonnaire ou adulte.
